(19) **Europäisches Patentamt**
**European Patent Office**
**Office européen des brevets**

(11) **EP 3 152 322 B1**

(12) **EUROPEAN PATENT SPECIFICATION**

(45) Date of publication and mention
of the grant of the patent:
**11.09.2019 Bulletin 2019/37**

(51) Int Cl.:
***G01N 33/72*** *(2006.01)*     ***G01N 33/80*** *(2006.01)*

(21) Application number: **15803598.0**

(86) International application number:
**PCT/US2015/034508**

(22) Date of filing: **05.06.2015**

(87) International publication number:
**WO 2015/188114 (10.12.2015 Gazette 2015/49)**

(54) **ESTIMATING RISK OF DEATH USING THE CLEARANCE VOLUME OF RED BLOOD CELLS AS BIOMARKER**

EINSCHÄTZUNG DES TODESRISIKOS UNTER VERWENDUNG DES CLEARANCE-VOLUMENS DER ROTEN BLUTKÖRPERCHEN ALS BIOMARKER

ESTIMATION DU RISQUE DE DÉCÈS UTILISANT LE VOLUME DE CLAIRANCE DES GLOBULES ROUGES COMME BIOMARQUEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.06.2014 US 201462007992 P**

(43) Date of publication of application:
**12.04.2017 Bulletin 2017/15**

(73) Proprietor: **The General Hospital Corporation Boston, MA 02114 (US)**

(72) Inventor: **HIGGINS, John M.**
**Cambridge**
**Massachusetts 02138 (US)**

(74) Representative: **Conroy, John et al**
**Fish & Richardson P.C.**
**Highlight Business Towers**
**Mies-van-der-Rohe-Straße 8**
**80807 München (DE)**

(56) References cited:
WO-A2-2012/037524     WO-A2-2014/074889
US-A1- 2007 172 956     US-A1- 2011 178 716
US-A1- 2011 190 143     US-A1- 2013 236 566
US-B2- 8 481 323

- J. M. HIGGINS ET AL: "Physiological and pathological population dynamics of circulating human red blood cells", PROCEEDINGS OF THE NATIONAL ACADEMY OF SCIENCES, vol. 107, no. 47, 8 November 2010 (2010-11-08), pages 20587-20592, XP055102295, ISSN: 0027-8424, DOI: 10.1073/pnas.1012747107
- HARSH H. PATEL ET AL: "Modulation of red blood cell population dynamics is a fundamental homeostatic response to disease : Modulation of red blood cell population dynamics", AMERICAN JOURNAL OF HEMATOLOGY, vol. 90, no. 5, 1 May 2015 (2015-05-01), pages 422-428, XP055425991, US ISSN: 0361-8609, DOI: 10.1002/ajh.23982
- C. KIM ET AL: "Association Between Iron Deficiency and A1C Levels Among Adults Without Diabetes in the National Health and Nutrition Examination Survey, 1999-2006", DIABETES CARE, vol. 33, no. 4, 12 January 2010 (2010-01-12), pages 780-785, XP055425993, US ISSN: 0149-5992, DOI: 10.2337/dc09-0836

**Description**

**FEDERALLY SPONSORED RESEARCH OR DEVELOPMENT**

This invention was made with Government support under K08DK083242 and DP2DK098087 awarded by the National Institutes of Health. The Government has certain rights in the invention.

**FIELD OF THE INVENTION**

The invention relates to methods for predicting morbidities associated with red blood cell volume variance.

**BACKGROUND**

A complete blood count (CBC), a widely used clinical test, summarizes basic features of circulating red blood cell (RBC) populations. The CBC can include measurement of the variation in volume of individual red blood cells (RBCs). The systems controlling the number, size, hemoglobin concentrations, and other characteristics of circulating human RBCs measured by the CBC are poorly understood. After release from the bone marrow, RBCs undergo reduction in both volume and total hemoglobin content by an unknown mechanism. In a healthy individual, after about 120 days, responding to an unknown trigger, the RBCs are typically removed or cleared.

Harsh H. Patel et al. describe in the American Journal of Hematology, vol. 90, no. 5, 1 May 2015, on pages 422-428 that the modulation of red blood cell population dynamics is a fundamental homeostatic response to disease.

**SUMMARY**

The present invention is defined by independent claim 1. The dependent claims depict additional embodiments of the invention.

The present invention is based, at least in part, on the discovery that a mechanism leading to an elevated RBC distribution width (RDW) in a patient includes a delay in *in vivo* clearance or removal of RBCs. Elevated RDW can serve as a biomarker for several adverse clinical situations, including but not limited to heart disease, pulmonary disease, sepsis, and cancer, complications in heart failure and severity of coronary artery disease, advanced stage and grade for many cancers, development of diabetes, chronic obstructive pulmonary disease, and stroke (See, e.g., References 1 to 8). RBC clearance rate is controlled in healthy individuals and is reduced or delayed in a range of adverse clinical situations. Because RBCs become smaller as they age, the delayed RBC clearance rate can correspond to a future increase in RDW. Thus, RBC clearance rate can also serve as a biomarker for the adverse clinical situations associated with elevated RDW. In some cases, the RBC clearance rate can further serve as a biomarker that predicts occurrence of the adverse clinical situations prior to normal presentation of the adverse clinical situations due to, for example, elevated RDW

The methods and systems described herein can include one or more of the following features. In some cases, the methods and systems include an operation of measuring a complete blood count (CBC) of a blood sample including a population of RBCs from a subject and using or generating a population dynamics model to estimate population dynamics of volume and hemoglobin content of the population of RBCs. The estimation of the population dynamics can include estimating an RBC clearance rate or estimating a parameter indicative of the RBC clearance rate based on data measured as part of the CBC. From the CBC, data representing single RBC volume and hemoglobin content of the RBCs in the blood sample can be used (e.g., aggregated) to determine the population dynamics.

The model for the population dynamics can include several parameters simulating various aspects of the population dynamics. In some examples, the estimated population dynamics can account for rates of hemoglobin reduction and volume reduction during lifecycles of the RBCs. The estimated population dynamics can further account for an initial fast phase and a subsequent slow phase of the lifecycles of the population of the RBCs. The estimated population dynamics may include a parameter indicative of a critical volume that, if reached by an RBC, will likely result in or coincide with the clearance of the RBC.

Based on the parameters estimated in the model for the population dynamics, the methods and systems can determine a treatment course for the subject. For example, in some cases, the critical volume can be associated with an RBC clearance delay as well as preceding elevated or increasing RDW. The present methods and systems therefore can predict, or be used to predict, that the subject has a risk of developing a morbidity associated with elevated RDW. Based on the estimation of various parameters, including the critical volume, the methods and systems can compare the estimated parameters to reference values for the parameters and determine a risk level that the subject will develop the morbidity associated with elevated RDW.

In another aspect, a method for evaluating a risk of death within 2 years for a subject includes measuring, using a hematology analyzer, a complete blood count (CBC) of a blood sample including a population of RBCs from a subject. In some embodiments, the method includes estimating a normalized clearance volume of the population of the RBCs based on the CBC. In some embodiments, the normalized clearance volume is indicative of a volume below which clearance occurs for most of the population of RBCs. In some embodiments, the method includes comparing the normalized clearance volume to a reference value. In some embodiments, the method further includes determining the risk of death for the patient within 2 years. In some embodiments, the risk of death is high if the normalized clearance volume is below the reference value, and the risk of death is low if the normalized clearance volume is above the reference value. In some embodiments, the method further includes selecting the patient for diagnostic workup if the risk of death is high.

In some implementations, estimating the normalized clearance volume of the population of the RBCs based on the CBC further can further include determining parameters indicative of each of a magnitude of variation in a rate of hemoglobin content reduction ($D_h$), a magnitude of variation in a rate of RBC volume reduction ($D_v$), an average rate of slow-phase RBC volume and hemoglobin content reduction ($\alpha$), an average rate of fast-phase RBC volume reduction ($\beta_v$), and an average rate of fast-phase RBC hemoglobin content reduction ($\beta_h$).

In some cases, estimating the normalized clearance volume can include computing a mean corpuscular hemoglobin concentration (MCHC) line from an MCHC of the CBC, and computing a probability of clearance of an RBC of the population of the RBCs based on the MCHC line. Measuring the CBC can include measuring a volume and hemoglobin content of the RBC. In some embodiments, the probability of clearance can be a function of the volume and the hemoglobin content. In some embodiments, the function can be a threshold function defined by at least the MCHC line. In some embodiments, the threshold function can be a threshold line perpendicular to the MCHC line such that, if the volume and the hemoglobin content of the RBC is less than the threshold line, the probability of clearance is 100%, and if the volume and the hemoglobin content of the RBC is greater than the threshold line, the probability of clearance is 0%.

In some examples, the reference value can be between 0.77 and 0.79. In some implementations, the reference value is between 0.7750 and 0.7950.

In some cases, measuring the CBC can include determining an RDW to be within a normal range.

In a further aspect, a non-transitory machine-readable storage device storing computer program instructions executable by a computer system to cause the computer system to perform operations includes receiving data representing a complete blood count (CBC) of a blood sample comprising a population of RBCs from a subject. In some embodiments, the operations include estimating a normalized clearance volume of the population of the RBCs based on the CBC. In some embodiments, the normalized clearance volume is indicative of a volume in which clearance occurs for most of the population of RBCs. In some embodiments, the operations include comparing the normalized clearance volume to a reference value. In some embodiments, the operations further include determining a risk level for the patient to develop the morbidity associated with elevated RDW within a specified time period. In some embodiments, the risk level is high if the normalized clearance volume is below the reference value, and the risk level is low if the normalized clearance volume is above the reference value. In some embodiments, the morbidity is not anemia.

In some implementations, estimating the normalized clearance volume of the population of the RBCs based on the CBC further can further include determining parameters indicative of each of a magnitude of variation in a rate of hemoglobin content reduction ($D_h$), a magnitude of variation in a rate of RBC volume reduction ($D_v$), an average rate of slow-phase RBC volume and hemoglobin content reduction ($\alpha$), an average rate of fast-phase RBC volume reduction ($\beta_v$), and an average rate of fast-phase RBC hemoglobin content reduction ($\beta_h$).

In some examples, the reference value can be between 0.77 and 0.79.

The methods and systems described herein have a number of advantages. In some embodiments, the estimated population dynamics can be easily determined using examinations that are regularly administered as part of a medical check-up, thus negating a need for special tests to screen for certain morbidities. In particular, the estimated population dynamics can be based on measurements taken as part of a CBC, which is a regularly administered test in clinics and hospitals. In some embodiments, the methods and systems thus render the CBC more useful for diagnostic purposes.

Furthermore, the methods and systems can (be used to) determine (or a healthcare professional can use the systems and methods to determine) that a subject may benefit from additional medical care before the subject develops life-threatening or uncomfortable symptoms and disorders. In some embodiments, the estimated population dynamics can help to determine that a subject has a high risk of developing a particular morbidity before the subject presents with the morbidity or comorbidities and symptoms thereof. A physician can thus administer preemptive care or select the patient for additional screening or diagnostic workup to reduce the risk that the subject will develop the morbidity. In some cases, the methods and systems can further determine a pathological condition that a CBC would be unable to detect by simply comparing traditional measurements of the CBC to reference values. In some embodiments, the healthcare professional can thus use the systems and methods to administer care to the subject before the onset of the condition or the manifestation of uncomfortable or progressive or advanced symptoms of the condition.

Unless otherwise defined, all technical and scientific terms used herein have the same meaning as commonly understood by one of ordinary skill in the art. Methods and materials described herein are for illustration purposes; other suitable methods and materials known in the art can also be used. The materials, methods, and examples are and not intended to be limiting. References parenthetically cited are listed herein below. In case of conflict between a reference mentioned herein and the present specification, the present specification, including definitions, will control.

Other features and advantages will be apparent from the following detailed description and figures, and from the claims.

## DESCRIPTION OF DRAWINGS

Fig. 1 is a block diagram of an exemplary diagnostic system.
Fig. 2 is a graph depicting a lifecycle of a population of red blood cells (RBCs).
Fig. 3 is a flow chart of an exemplary process to select a patient for diagnostic workup.
Fig. 4 is a schematic diagram of an exemplary computer system.
Fig. 5A is a graph that schematically depicts an exemplary model of population dynamics of a population of RBCs.
Fig. 5B is a graph that schematically depicts an exemplary normalized clearance volume of the model of Fig. 5A.
Fig. 5C is a graph that schematically depicts a probability distribution of clearance predicted by the model of Fig. 5A.
Figs. 6A to 6E are exemplary histograms of parameters related to a population of RBCs.
Fig. 7 shows box plots of exemplary parameters related to a population of RBCs.
Figs. 8A to 8B are 3D graphs of exemplary probability density functions for clearance of an RBC from a population of RBCs based on a volume and hemoglobin content of the RBC.
Fig. 8C show graphs of the exemplary probability density functions of Figs. 8A to 8B normalized to a mean cell volume of the population of RBCs.
Figs. 9A to 9D are plots showing correlations between RDW and parameters related to a population of RBCs.
Fig. 10 is an exemplary box plot showing glycated hemoglobin fraction for two populations of subjects.
Fig. 11 is an exemplary box plot showing ferritin levels for two populations of subjects.

## DETAILED DESCRIPTION

Red blood cells (RBCs) are removed or cleared from circulation through a poorly-understood process that can become altered in states of disease. These alterations can lead to various RBC population characteristics in diseased patients that differ from RBC population characteristics in healthy patients. For example, if a particular cellular characteristic, like volume or hemoglobin concentration, changes during the course of a RBC's time in the circulation, the average speed of this change across the population of RBCs from a patient with some diseases can be different from the average speed of this change across the population of RBCs from patients without the disease.

Population dynamics of RBCs is one example of an aspect of the RBC population characteristics that may change due to disease. In human adults, a flux of RBCs is characterized by a net clearance or increase in the number of RBCs. New RBCs enter the circulation of a healthy human adult at a rate (herein also referred to as a flux of RBCs) of more than 2 million per second. The flux can be characterized by RBC maturation and clearance processes. Each RBC can circulate for about 100-120 days, with its volume decreasing by about 30% during that time from an initial ~115 fl to a final ~80 fl. The major intracellular RBC constituent is hemoglobin, and the typical RBC's hemoglobin mass drops by about 20% from an initial ~35 pg on day 1 to a final ~28 pg 100-120 days later. The RBC hemoglobin concentration increases slightly and is tightly regulated to ensure efficient oxygen delivery to the tissues.

While the molecular and cellular mechanisms controlling these maturation and clearance processes are not fully understood (See, e.g., Reference 14), the net effects of these mechanisms have been examined using RBC labeling and other studies (See, e.g., Reference 10 and 12 to 16). The circulating population of RBCs is thus continuously changing. In healthy individuals (and patients with mild disease) the characteristics of the population can be stable. These characteristics described may change due to mechanisms leading to or associated with pathological conditions, and analysis of these characteristics can be used to predict a likelihood of a subject to develop the pathological conditions. In some cases, the characteristics described may change due to the presentation of the pathological conditions before other biomarkers associated with those pathological conditions change to indicate presentation. These characteristics thus can allow for early diagnosis.

As many pathological conditions are associated with elevated RDW, population dynamics (e.g., hemoglobin and volume dynamics) that are associated with mechanisms leading to elevated RDW can be estimated to predict onset of the pathological conditions before any change in RDW is seen. RDW is a typical attribute of a population of RBCs that is measured using the CBC. RDW measures an amount of variation in volumes of RBCs in the population of the RBCs. RDW is represented mathematically as RDW = $\frac{\sqrt{\text{variance}(V)}}{\text{mean}(V)}$, where $V$ represents the set of volumes of a population of RBCs. RDW's mathematical representation shows that an increase in RDW can be attributed to an increase in RBC volume variance, a decrease in mean cell volume (MCV), or a combination thereof.

Triggers of an increase in RBC volume variance might be categorized based on when the triggers occur during the RBC lifecycle. For example, the triggers might include an increase in reticulocyte volume variance (rRDW) (See, e.g., Reference 18) while the reticulocytes are exiting the bone marrow, an increase in the accumulation of volume variation during RBC maturation in the circulation, or a delay in the clearance of senescent RBCs.

Thus, an increase in RDW might be attributable to one of the following:

1) decreased MCV,
2) increased reticulocyte volume variance,
3) increased heterogeneity in the rate of RBC volume reduction occurring in the peripheral circulation,
4) delayed RBC clearance, or
5) a combination thereof.

Since elevated RDW is associated with many pathological conditions, each of the above possibilities may play a role in mortality and morbidity risk. In some studies, a possible role for MCV in mortality and morbidity risk has been assessed to identify RDW associations, and these studies have determined MCV to be unlikely to be the dominant factor in RDW elevation (See, e.g., References 1 to 8). In other studies, rRDW has been determined not to be narrowly distributed in healthy people, fluctuating with a coefficient of variation of more than 8%. Heterogeneity of the volume reduction processes occurring while RBCs circulate, and the rate of RBC clearance, can be associated with elevated RDW and are thus assessed using the present systems and methods.

**Overview of Systems and Methods**

The systems and methods described herein can be implemented to identify the presence of factors associated with risk of developing elevated RDW to predict or detect presentation of pathological conditions causing the elevated RDW before the RDW is elevated in a subject. One or more of a doctor, physician assistant, nurse, lab technician, or other medical or healthcare professional or personnel, or a combination thereof (generically referred to both individually and collectively herein as healthcare professionals) can use a diagnostic system 100 to measure and analyze a sample of blood from the subject, estimate an RBC flux based on a rate of RBC clearance, and determine a risk level of the subject to develop a disease associated with elevated RDW, e.g., before elevated RDW develops. The sample of blood includes a population of RBCs for which the diagnostic system 100 can generate relevant population characteristics data. The healthcare professional can use the diagnostic system 100 to perform analyses on these data to generate additional parameters associated with the population characteristics. The diagnostic system 100 includes a hematology analyzer 105 that the healthcare professional can use to collect data from the sample of blood and a computing system 110 to receive and analyze the data. In some implementations, the diagnostic system 100 includes a hemoglobin testing system 115 and an immunoassay analyzer 120. The diagnostic system 100 can also include a memory storage element 125 that can store data pertaining to, for example, normal ranges of measurements taken from the hematology analyzer 105, the hemoglobin testing system 115, and the immunoassay analyzer 120.

The hematology analyzer 105 can be used to perform a complete blood count (CBC) on the blood sample to determine characteristics about the population of the RBCs. The CBC, which is a widely used clinical test that can guide the diagnosis and treatment of many diseases, summarizes basic characteristics of circulating RBC populations. The basic characteristics can include the RDW, which quantifies a coefficient of variation of the volume of individual RBCs. The CBC also measures population characteristics such as a volume fraction of cells in the blood sample (hematocrit), the MCV, and the mean intracellular hemoglobin mass (MCH). By performing the CBC, the hematology analyzer 105 can generate data indicative of hematocrit, MCV, RDW, MCH, volumes of each of the RBCs in the population, and hemoglobin content of each of the RBCs in the population.

In some cases, the RDW determined by the hematology analyzer 105 can be indicative of, as described herein, an increased risk for a morbidity and/or mortality, such as, for example, all-cause mortality, mortality from heart disease, pulmonary disease, sepsis, and cancer; complications in heart failure and severity of coronary artery disease; advanced stage and grade for many cancers; development of diabetes, chronic obstructive pulmonary disease, stroke, anemia, and more (See, e.g., References 1 to 8). In some cases, the RDW can be used to distinguish causes of anemia such as iron deficiency (See, e.g., References 8 and 17). Elevated RDW can be associated with an increased risk of poor prognosis in heart failure and coronary artery disease (See, e.g., References 2 and 4).

While the diagnostic system 100 can use RDW as a biomarker to inform clinical practice and diagnosis and can treat RDW as a biomarker to understand early stages of fundamental disease processes, the diagnostic system 100 can use other biomarkers that precede and predict the presence or development of initially elevated or further elevated RDW in the subject. In some implementations, the computing system 110 can use a population dynamics model to estimate a delay in the *in vivo* clearance of RBCs, and the healthcare professional can use the estimated clearance delay to make clinical recommendations for the subject. The RBC clearance rate can be controlled in healthy individuals but can be reduced in a range of adverse clinical situations. The estimated RBC clearance delay can thus serve as a biomarker or indicator for certain adverse clinical situations for the patient. A healthcare professional, or, in some cases, the computing system, can evaluate the estimated RBC clearance delay to determine a likelihood that the patient develops a pathology or to determine a score indicating the likelihood that the patient develops the pathology.

An RBC clearance delay can lead to an elevated RDW. Because RBCs become smaller as they age, the RBC clearance delay can expand the low-volume tail of the volume distribution and increase RDW. Thus, the healthcare professional can use the computing system 110 to estimate the RBC clearance delay and use the estimated RBC clearance delay as a biomarker for morbidities associated with (risk of) development of elevated RDW. In some cases, based on the estimated RBC clearance delay, the healthcare professional can use the computing system 110 to determine that latent or incipient pathology exists. The estimated RBC clearance delay can precede, for example, an elevation in RDW and a future presentation of RDW-associated pathologic conditions. Risk of all-cause anemia can be one condition associated with elevated RDW (See, e.g., References 5 and 6). In some implementations, the estimated RBC clearance delay can indicate a present pathological condition that the CBC cannot typically detect.

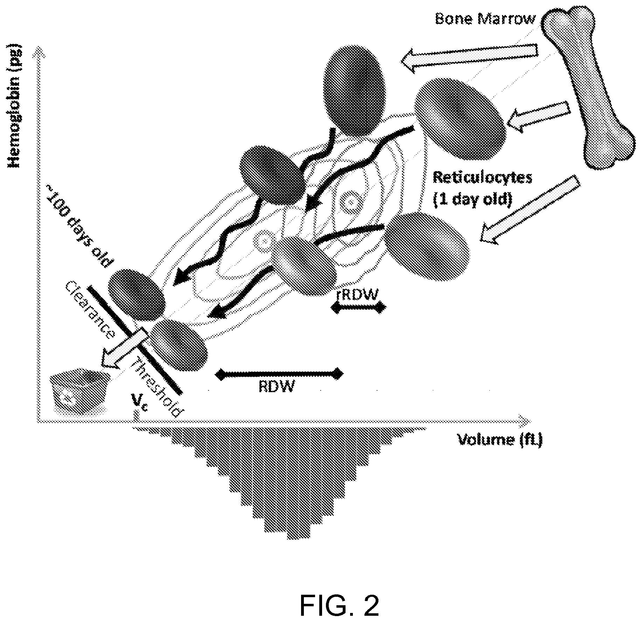

In one example, the healthcare professional can use the computing system 110 to generate parameters to define a model as depicted in Fig. 2, which shows an example schematic of RBC population dynamics of RBCs of a healthy individual and determinants of volume variation. Reticulocytes can enter the circulation from bone marrow, as shown in the top right. The computing system 110 can determine a volume and hemoglobin distribution of reticulocytes in the healthy individual, shown as contours at the top right. Each of the reticulocyte contours enclose 75%, 50%, and 25% of reticulocytes. The healthcare professional can further use the computing system 110 to determine the means of the volume and the hemoglobin of the reticulocytes in the healthy individual. The center of the reticulocyte contours indicates the means.

By implementing an example of the population dynamics model described herein, the computing system 110 can determine simulated trajectories of the RBCs, which are depicted in Fig. 2 as black arrows. The computing system 110 can further determine the healthy individual's total RBC volume and hemoglobin distribution, which is shown as contours to the left of the reticulocyte contours. The total RBC contours enclose 90%, 75%, 50%, and 25% of cells, with means indicated by the circle at the center of the total RBC contours.

Senescent RBCs can be cleared and recycled before reaching, for example, a threshold volume and hemoglobin that depends on the cell's volume and hemoglobin. The computing system 110 can determine the threshold by, in part, computing a normalized critical volume ($v_c$) (shown in bottom left of Fig. 2) indicative of the volume at which most of the RBCs are cleared. The normalized critical volume $v_c$ can, in part, define a clearance threshold indicative of the volume and hemoglobin at which most of the RBCs are cleared. An example process of computing the normalized critical volume ($v_c$) is described herein. The computing system 110 can define the hemoglobin of the threshold to be the hemoglobin concentration of a typical RBC having a typical hemoglobin concentration that is equal to the mean hemoglobin concentration of the population. The normalized critical volume ($v_c$) can serve an indicator of RBC clearance delay, as described in detail in Example 2. Therefore, the normalized critical volume ($v_c$) can be computed to determine if a subject presents a pathological condition that the CBC cannot typically detect. See also WO2012/037524.

The variation in the RBC volume distribution, shown as a histogram in a lower portion of Fig. 2, can be quantified by its coefficient of variation, called the red cell distribution width or RDW. In the example simulated population dynamics shown in Fig. 2 and described in more detail in Example 1, the RDW can be determined by, for example, the mean volume, the coefficient of variation in reticulocyte volume (rRDW), the degree of heterogeneity in volume dynamics (black arrows) during maturation, and the location of a threshold line corresponding to the normalized clearance volume ($v_c$).

In addition to the parameters measured using the CBC or generated as part of the models and simulations described herein, the diagnostic system 100 can include the hemoglobin testing system 115 to measure amounts of different types of hemoglobin present in the population of RBCs. The hemoglobin testing system 115 can measure, for example, hemoglobins A1a, A1b, LA1c, A1c, P3, P4, and Ao. The hemoglobin testing system 115 can measure a glycated hemoglobin (hemoglobin A1c or HbAlc) fraction.

The computing system 110, based on the glycated hemoglobin fraction measured by the hemoglobin testing system 115, can determine an age of the population of the RBCs. The age can be a mean RBC age of the population of the RBCs. In some examples, a fraction of the population of RBCs above a threshold age can be indicative of the age of the population of the RBCs.

The glycated hemoglobin fraction can be proportional to the mean plasma glucose concentration and to the mean RBC age (See, e.g., References 20 to 22). In non-diabetic individuals, the glycated hemoglobin fraction can be linearly dependent on mean RBC age and therefore can serve as a parameter indicative of the mean RBC age (See, e.g., References 14, 20, and 23 to 27) or indicative of the presence of an expanding fraction of the population of the RBCs that includes oldest RBCs. The parameter can be indicative that the fraction including the oldest RBCs is expanding or is not expanding, thereby serving as a binary indicator of an expanding fraction. In some cases, the parameter can be indicative of a value for the fraction including the oldest RBCs that is expanding. The hemoglobin testing system 115 can measure the hemoglobin content of the population of RBCs and generate data indicative of the hemoglobin content, including data indicative of the glycated hemoglobin fraction of the population of RBCs. The computing system 110 can thus, in some implementations, further estimate a mean RBC age of the population of RBCs and evaluate the risk level of the subject to develop pathological conditions associated with elevated RDW. The computing system 110 can receive the data indicative of the glycated hemoglobin fraction and then can estimate the mean RBC age using the data.

In one example, the mean glycated hemoglobin fraction across the total population of RBCs in a typical healthy person can be about 5.3%. The youngest cells have a glycated fraction of 0%, and the oldest have a glycated hemoglobin fraction of about 10.6%. For a uniform distribution of ages, the average is 5.3%. The hemoglobin in individual RBCs can become glycated over time, and the increase can be substantially linear in time. If clearance is delayed, then some RBCs will exhibit glycated hemoglobin fractions greater than 10.6%. The presence of these cells will increase the average glycated fraction above 5.3%. Using the glycated hemoglobin fraction, the computing system can detect the increase in average glycated hemoglobin fraction.

In some examples, the computing system can determine the presence of RBCs with glycated hemoglobin fractions above a threshold percent. RBCs having a glycated hemoglobin fraction above the threshold percent can be considered the oldest RBCs. The oldest RBCs can be defined as those with glycated hemoglobin fractions greater than the threshold percent, such as, for example, 9% to 11%, 10% to 10.5%, 10.5% to 11%, or greater. The threshold age for the oldest RBCs can be, for example, 90 days to 130 days, 100 days to 120 days, or 130 days or more. The computing system 110 then can determine a fraction or percent of the whole population of RBCs that are oldest RBCs. In another example, an expanding fraction including the oldest RBCs can be identified by comparing the percentage of oldest RBCs (e.g., those with glycated hemoglobin fractions greater than threshold percentages described herein) to a reference range or to a subject's prior measurements for glycated hemoglobin fraction. For example, a typical healthy person with a mean glycated fraction of 5.3% can be expected to have about 13% of all RBCs with glycated hemoglobin fractions greater than 9%. If more than 13% of the RBCs have glycated fractions greater than 9%, the computing system 110 can determine that the subject has the fraction including the oldest RBCs is expanding. Thus, the computing system 110 can compare the fraction of all RBCs having glycated hemoglobin fractions greater than the threshold percent described herein (e.g., the oldest RBCs) to a reference value that is between, for example, 13% and 15% (e.g., the typical percent of the population of RBCs that are considered the oldest RBCs). If the fraction is greater than the reference value, the parameter can indicate a presence of an expanding fraction of the oldest RBCs. If the fraction is below the reference value, the parameter can indicate an absence of an expanding fraction of the oldest RBCs. The expanded fraction can be expected generally to be associated with an increase in the average glycated hemoglobin fraction across the total RBC population as well as the average mean RBC age across the total population of RBCs.

The computing system 110 can estimate the mean RBC age relative to a baseline RBC age. Alternatively or additionally, the computing system 110 can estimate an absolute mean RBC age of the population of RBCs in the blood sample. To estimate the absolute mean RBC age of the population of RBCs, the RBCs can be labelled with, for example, biotin.

Elevated mean RBC age can precede elevated RDW and be associated with RBC clearance delay. The RBC clearance delay described herein can increase RDW and also enable cells to circulate beyond the time when the RBCs would ordinarily have been cleared in a healthy individual. The RBC clearance delay can thus increase the average RBC age and lifespan. The mean RBC age can serve as a biomarker for morbidities associated with elevated RDW. The computing system 110 can compare the mean RBC age of the subject to mean RBC age of typical healthy subjects. Based on the comparison, the computing system 110 can determine the risk level for the subject to develop morbidities associated with elevated RDW.

In some implementations, the diagnostic system 100 can determine that the subject presents or is likely to develop pathological conditions, such as anemia, associated with elevated RDW. Using appropriate assays, the immunoassay analyzer 120 can measure ferritin levels in the blood sample of the subject and generate data indicative of the ferritin levels. The computing system 110 can receive the data indicative of the ferritin levels in the blood sample of the subject. Based on the data indicative of the ferritin levels, the diagnostic system 100 can determine that the subject has, for example, iron deficiency anemia or iron deficiency with incipient anemia.

In some cases, the data indicative of the ferritin levels may indicate that the subject does not have iron deficiency, anemia, or other pathological conditions related to decreased ferritin levels. The estimated RBC clearance can be normal or abnormal (e.g., delayed). In the case where the estimated RBC clearance is delayed, the computing system 110 may determine that-although the subject may or may not present with the pathological conditions associated with the decreased ferritin levels-the estimated RBC clearance delay indicates a risk of the subject to develop a pathological condition.

The memory storage element 125 can include reference values indicative of normal ranges for the measurements taken by, for example, the hematology analyzer 105, the hemoglobin testing system 115, and the immunoassay analyzer 120. The reference values can serve as thresholds to which measurements taken from the subject can be compared to determine if the subject has a risk of developing a morbidity. For example, the memory storage element 125 can include normal ranges for measurements of the CBC taken by the hematology analyzer 105, as shown in Table 1 below. The values for normal ranges may vary depending on the clinical laboratory that defines the normal range.

Table 1: Normal Ranges for Complete Blood Counts

| TEST (units) | POPULATION | NORMAL RANGE |
|---|---|---|
| RBC (millions/mm$^3$) | ADULT MALES >18 YEARS | 4.50-5.90 |
| | ADULT FEMALES >18 YRS | 4.00-5.20 |
| | MALE 12-18 YEARS | 4.50-5.30 |
| | FEMALE 12-18 YEARS | 4.10-5.10 |
| HGB (g/dl) | ADULT MALES >18 YEARS | 13.5-17.5 |
| | ADULT FEMALES >18 YRS | 12.0-16.0 |
| | MALE 12-18 YEARS | 13.0-16.0 |
| | FEMALE 12-18 YEARS | 12.0-16.0 |
| HCT (%) | ADULT MALES >18 YEARS | 41.0-53.0 |
| | ADULT FEMALES >18 YRS | 36.0-46.0 |
| | MALE 12-18 YEARS | 37.0-49.0 |
| | FEMALE 12-18 YEARS | 36.0-46.0 |
| MCV (fl) | ALL ADULTS >18 YEARS | 80-100 |
| | MALE 12-18 YEARS | 78-98 |
| | FEMALE 12-18 YEARS | 78-102 |
| MCH (pg/RBC) | ADULT >18 YEARS | 26.0-34.0 |
| | 12-18 YEARS | 25.0-35.0 |
| MCHC (g/dl) | ALL >2 YEARS | 31.0-37.0 |
| RDW (%) | ALL | 11.5-14.5 |

The memory storage element 125 can further include normal ranges for, for example, ferritin levels and HbAlc levels measured by the immunoassay analyzer 120 and the hemoglobin testing system 115, respectively, as shown in Table 2 below.

Table 2. Normal Ranges for Other Clinical Laboratory Tests

| TEST (units) | POPULATION | NORMAL RANGE |
|---|---|---|
| Glucose (mg/dl) | ALL | 70 - 110 |
| Hemoglobin A1c (%) | ALL | 3.8 - 6.4 |
| Ferritin (ng/ml) | ADULT MALE | 30-300 |
| | ADULT FEMALE | 10-200 |

The memory storage element 125 can include odds ratios for future pathological conditions, such as anemia, for parameters measured by the hematology analyzer 105. As shown in Table 3 below, the memory storage element 125 can include odds ratios for a specific condition, such as anemia, for each of the parameters measured by the hematology analyzer 105.

Table 3. Odds Ratios of Future Anemia for Thresholds of CBC parameters

| Predictor | Threshold | Odds Ratio for Future Anemia | 95% Confidence Interval | |
|---|---|---|---|---|
| RDW | 12.25 | 0.9 | 0.3 | 2.7 |
| | 12.50 | 1.1 | 0.6 | 2.2 |
| | 12.75 | 1.3 | 0.8 | 2.3 |
| | 13.00 | 1.9 | 1.2 | 3.0 |
| | 13.25 | 2.0 | 1.2 | 3.2 |
| | 13.50 | 1.9 | 1.1 | 3.3 |
| | 13.75 | 2.0 | 1.1 | 3.8 |
| | 14.00 | 2.3 | 0.9 | 5.2 |
| | 14.25 | 2.1 | 0.7 | 6.1 |
| HGB | 12.5 | 0.8 | 0.3 | 2.1 |
| | 13.0 | 1.2 | 0.7 | 2.1 |
| | 13.5 | 0.9 | 0.6 | 1.4 |
| | 14.0 | 0.9 | 0.6 | 1.4 |
| | 14.5 | 0.7 | 0.4 | 1.1 |
| | 15.0 | 0.5 | 0.3 | 1.0 |
| MCHC | 32.5 | 1.2 | 0.5 | 3.0 |
| | 33.0 | 1.4 | 0.8 | 2.4 |
| | 33.5 | 1.1 | 0.7 | 1.7 |
| | 34.0 | 0.9 | 0.5 | 1.5 |
| | 34.5 | 1.0 | 0.6 | 1.9 |
| | 35.0 | 0.9 | 0.4 | 2.2 |
| MCH | 28.0 | 2.7 | 0.8 | 9.1 |
| | 29.0 | 1.2 | 0.6 | 2.3 |
| | 30.0 | 1.3 | 0.8 | 2.1 |
| | 31.0 | 1.2 | 0.8 | 2.0 |
| | 32.0 | 0.7 | 0.4 | 1.3 |
| | 33.0 | 0.6 | 0.2 | 1.5 |
| MCV | 80.0 | 0.3 | 0.0 | 5.3 |
| | 83.0 | 0.7 | 0.2 | 2.4 |
| | 86.0 | 1.4 | 0.6 | 3.1 |
| | 89.0 | 1.7 | 0.9 | 3.0 |
| | 92.0 | 1.1 | 0.7 | 1.8 |
| | 95.0 | 1.2 | 0.7 | 2.1 |

(continued)

| Predictor | Threshold | Odds Ratio for Future Anemia | 95% Confidence Interval | |
|---|---|---|---|---|
| HCT | 38.0 | 1.0 | 0.5 | 1.9 |
| | 40.0 | 0.9 | 0.5 | 1.4 |
| | 42.0 | 0.8 | 0.5 | 1.2 |
| | 44.0 | 0.8 | 0.5 | 1.4 |
| | 46.0 | 0.5 | 0.2 | 1.5 |
| | 48.0 | 0.6 | 0.1 | 5.2 |

The memory storage element 125 can include reference values for thresholds corresponding to odds ratios for future anemia for estimated parameters if the estimated parameters cross the thresholds. As shown in Table 4, the memory storage element 125 can include odds ratios for various threshold values of the normalized critical volume ($v_c$). An example process of computing the normalized critical volume ($v_c$), which is correlated with and can therefore indicate RBC clearance delay, is described herein. In the case of the normalized critical volume ($v_c$), an estimated critical volume from the subject above the threshold are considered normal and can therefore indicate that the subject does not have delayed RBC clearance. An estimated critical volume below the threshold is considered abnormal and can indicate that the subject has delayed RBC clearance. See also WO2012/037524.

Table 4. Odds Ratios of Future Anemia for Thresholds of CBC parameters and $v_c$

| Predictor | Threshold | Odds Ratio for Future Anemia | 95% Confidence Interval | |
|---|---|---|---|---|
| $v_c$ | 0.7750 | 8.5 | 1.1 | 63.4 |
| | 0.7775 | 6.2 | 1.5 | 26.1 |
| | 0.7800 | 5.6 | 1.7 | 18.5 |
| | 0.7825 | 3.7 | 1.5 | 8.8 |
| | 0.7850 | 3.1 | 1.5 | 6.4 |
| | 0.7875 | 2.1 | 1.2 | 3.7 |
| | 0.7900 | 1.8 | 1.1 | 3.1 |
| | 0.7925 | 1.7 | 1.0 | 2.7 |
| | 0.7950 | 1.5 | 0.9 | 2.3 |

The computing system 110 can access the memory storage element 125 and compare the measurements from each of the hematology analyzer 105, the hemoglobin testing system 115, and the immunoassay analyzer 120 against the data indicative of normal ranges for each of the measurements (e.g., the data shown in Tables 1 to 2). From the comparison, the computing system 110 can determine that the measurements of the blood sample falling outside of the normal ranges can indicate presence of a pathological condition. In other cases, the healthcare professional can determine from the measurements a likelihood the subject will develop a morbidity, risk of death, or a score indicating a likelihood that the subject will develop a morbidity or will die within a specified period of time.

The computing system 110 can further compare measurements (e.g., taken from each of the hematology analyzer 105, the hemoglobin testing system 115, and the immunoassay analyzer 120) and parameters (e.g., estimated by the computing system 110) to threshold values stored on the memory storage element 125 (e.g., the data shown in Table 3 and 4). The computing system 110, based on the comparison, can determine that the measurements and estimated parameters cross (e.g., fall below or exceed) the respective threshold values. The measurements and the estimated parameters that cross their respective threshold values can indicate a corresponding odds ratio. The odds ratio indicates a likelihood that a pathological condition (e.g., anemia as shown in Tables 3 and 4) will occur when the estimated parameters and measurements cross their respective thresholds. The computing system 110 can thus predict the likelihood that a pathological condition can occur based on the estimated parameters and the measurements.

**Selecting a Subject for Diagnostic Workup**

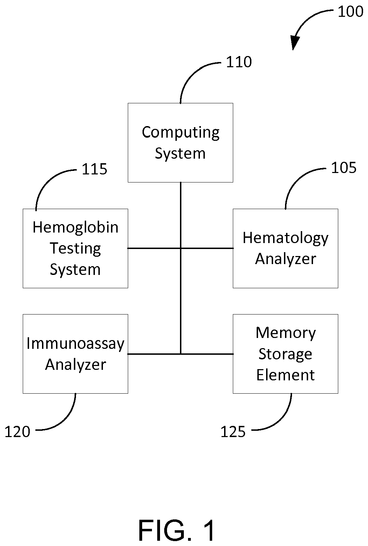

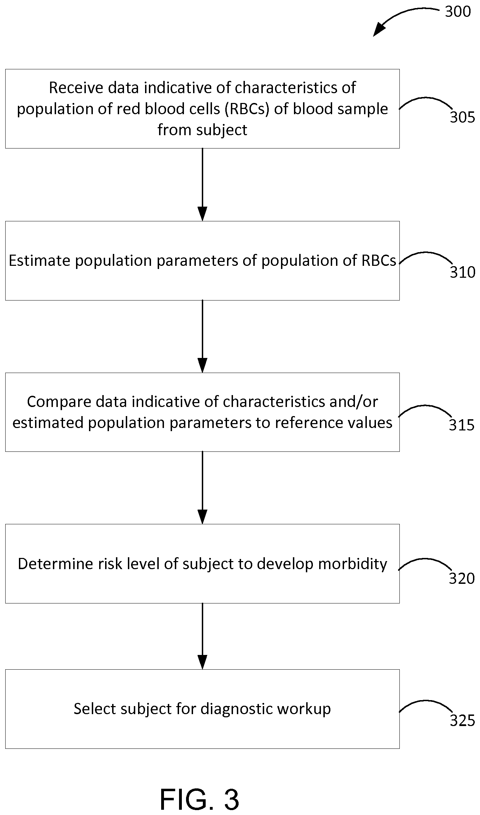

Using the systems and methods described herein, in one example, a computing system (e.g., the computing system 110 of Fig. 1), a diagnostic system, and other appropriate systems can be used to implement at least part of a process 300 to select a subject for diagnostic workup for a morbidity associated with elevated RDW, as shown in a flow chart depicted in Fig. 3. In some examples, a healthcare professional, physician, or other appropriate medical professional can implement operations of the process 300.

At operation 305, the healthcare professional can use the computing system to receive data indicative of measurements of a population of RBCs in a blood sample from a subject. The data can include CBC data. The data can be indicative of ferritin levels, hemoglobin content, glycated hemoglobin content, glucose content, and other parameters associated with the population of the RBCs. The CBC data can include data indicative of, for example, cell volume of each of the RBCs, hemoglobin content of each of the RBCs, MCV, MCH, RDW, rRDW, and other population statistics. The CBC data can be generated by a hematology analyzer (e.g., the hematology analyzer 110 of Fig. 1). The data can be generated by other testing devices and analyzers, such as chromatography assays, hemoglobin testing systems (e.g., the hemoglobin testing system 115 of Fig. 1), immunoassay analyzers (e.g., the immunoassay analyzer 120 of Fig. 1), and other appropriate systems to analyze the population of RBCs in the blood sample. In some cases, the computing system can receive data indicative of measurements of reticulocytes of the population of RBCs.

At operation 310, the healthcare professional can use the computing system to estimate population parameters based on the data received at the operation 305. The population parameters can be indicative of a flux of RBCs. The population parameters can be indicative of population dynamics of hemoglobin content and volume of the population of the RBCs. The population parameters can be indicative of different phases of lifecycles of the RBCs, such as, for example, a slow phase of hemoglobin and volume reduction and a fast phase of hemoglobin and volume reduction. In some implementations, the population parameters can include values that define a sigmoid function, a threshold function, or other mathematical function that characterizes the population dynamics of the population of the RBCs. The function can characterize a critical volume below which most RBCs are cleared from the population of the RBCs. To estimate the population parameters, the computing system can be used to identify an optimal parameter set based on the data received at the operation 305. The computing system can be used to perform a least-squares fit on functions including the population parameters to estimate the population parameters. In some cases, the population parameters include at least one of a magnitude of variation in a rate of hemoglobin content reduction ($D_h$), a magnitude of variation in a rate of RBC volume reduction ($D_v$), a normalized clearance volume ($v_c$) indicative of a volume in which clearance occurs for most of the population of RBCs, an average rate of slow-phase RBC volume and hemoglobin content reduction ($\alpha$), an average rate of fast-phase RBC volume reduction ($\beta_v$), and an average rate of fast-phase RBC hemoglobin content reduction ($\beta_h$).

In some examples, the computing system may be used to implement a dynamic model as described in Example 1 herein. In some examples, the population parameters can be indicative of a mean RBC age. The healthcare professional can alternatively or additionally perform an operation to determine a glycated hemoglobin fraction of the subject. For example, the healthcare professional can use a hemoglobin testing system to measure the glycated hemoglobin fraction. Based on the glycated hemoglobin fraction, the healthcare professional can estimate the mean RBC age of the population of the RBCs or the presence of an expanding fraction of oldest RBCs. The mean RBC age can be linearly proportional to the glycated hemoglobin fraction.

At operation 315, the computing system can be used to compare the estimated population parameters and/or the data indicative of measurements of the population of the RBCs to reference values. The reference values can define normal ranges (e.g., the normal ranges shown in Tables 1 and 2) for the estimated population parameters or the data indicative of the measurements of the population of the RBCs. During the operation 315, the computing system 315 can determine that each of the estimated population parameters and/or the data indicative of the measurements is either (i) within the normal range (e.g., the estimated population parameter and/or the measurement is normal) or (ii) outside of the normal range (e.g., the estimated population parameter and/or the measurement is abnormal). The references values can be stored on a memory storage element (e.g., the memory storage element 125 of Fig. 1). The reference values can be normal ranges or standard values typically found in patients in a clinical setting. In some cases, the reference values may include averages, standard deviations, and other population statistics found in a normal population of patients. The reference values can be lower and upper limits of ranges for the characteristics or the estimated population parameters. In some cases, the reference values can be threshold values (e.g., the threshold values shown in Tables 3 and 4) that are each one of a lower limit or an upper limit for the characteristics or the estimated population parameters of the population of the RBCs. Examples of reference values that can be stored on the memory storage element 125 are shown in Tables 1 to 4 included herein. While the computing system has been described to compare the estimated population parameter to the reference value, in some example, a healthcare professional can make the comparison.

In some cases, the estimated population parameter is a normalized critical volume ($v_c$), as described herein. The computing system can be used to determine a population MCHC by calculating a distribution of volume and hemoglobin content values for each of the RBCs in the population of RBCs in the sample. The computing system can be used to calculate a transformation of each cell's volume and hemoglobin on the MCHC line. The computing system can be used to further determine a fraction of RBCs in the sample whose transformations fall below a threshold percentage of the mean transformation to provide a sample fraction. In some cases, the reference value for the normalized critical volume is between, for example, 0.7750 and 0.7950 or 0.77 and 0.79. The reference value can be selected using methods known in the art and can represent values that maximize sensitivity and specificity. See also WO2012/037524.

In some examples, the estimated population parameter is the mean RBC age, and the healthcare professional can compare the mean RBC age of the subject to a reference value indicative of a normal range or normal value for the mean RBC age in healthy individuals. In some cases, the reference value for the mean RBC age is between, for example, 38 days and 60 days, 48 days and 52 days, or 45 days or 55 days.

In some examples, the estimated population parameter is a fraction of the population of the RBCs having an RBC age above a threshold age. The fraction can be an oldest RBC fraction that indicates the fraction of the population of the RBCs that can be considered the oldest RBCs. The threshold age for the oldest RBCs can be, for example, 90 days to 130 days, 100 days to 120 days, or 130 days or more. The fraction can be compared to a reference value indicative of a normal fraction of the population of the RBCs that are considered the oldest RBCs. The reference value can be, for example, 13% to 17% or 13% to 15%

At operation 320, based on the comparison performed at the operation 315, the healthcare professional can determine a risk level of the subject to develop a morbidity. In some cases, the healthcare professional uses a computing system to determine the risk level. The computing system and/or the healthcare professional can determine a likelihood of the subject to develop the morbidity or a score indicating the likelihood of the subject to develop the morbidity. The morbidity can be associated with elevated RDW. In some examples, from the CBC data, the subject does not have elevated RDW. The morbidity can be, for example, heart disease, pulmonary disease, sepsis, and cancer, complications in heart failure and severity of coronary artery disease, advanced stage and grade for many cancers, development of diabetes, chronic obstructive pulmonary disease, stroke, and anemia. The risk level can be represented as an odds ratio. The odds ratio can be defined as a ratio of a likelihood that the subject develops the morbidity during a specified period of time due to presence of an abnormality to a likelihood that the subject does not develop the morbidity during the specified period of time due to absence of the abnormality. The specified period of time can be, for example, within 1 month, 2 months, 3 months, or more. In some cases, the specified period of time can be between 0 months and 3 months, 3 months and 6 months, 6 months and a year. In some implementations, the risk level may be a determination, in which the risk level is high or low. In other implementations, the risk level may vary within a range of values, such as a numeric odds ratio. In the case of the normalized critical volume ($v_c$), the odds ratio can vary between, for example, 1.5 and 8.5 (e.g., 1.7 and 8.5, 1.8 and 8.5, 2.1 and 8.5, 3.1 and 8.5, etc.). In some cases, the computing system and/or the healthcare professional can assess the risk level based on comparing several estimated parameters and/or several measurements to their respective reference values, and in some cases, the computing system and/or the healthcare professional can assess the risk level based on one estimated parameter or one measurement.

At operation 325, based on the risk level determined at the operation 320, the healthcare professional can select the subject for diagnostic workup associated with the morbidity. In some cases, the risk level or the odds ratio representative of the risk level may be low, and the healthcare professional can determine that the diagnostic workup does not include diagnostic procedures for the morbidity. In some cases, the risk level or the odds ratio representative of the risk level may be high, and the healthcare professional can determine that the diagnostic workup should include diagnostic procedures for the morbidity. For the case of the normalized clearance volume described herein, the healthcare professional can determine a risk level for the patient to develop the morbidity associated with elevated RDW within a specified time period based on comparing the normalized clearance volume to its reference value or threshold. The healthcare professional can determine the risk level to be high if the normalized clearance volume is below the reference value and to be low if the normalized clearance volume is above the reference value. The healthcare professional can then select the patient for diagnostic workup for the morbidity if the risk level is high. The diagnostic workup can include additional blood examinations, physical examinations, or other appropriate tests to determine presentation of the morbidity or risk of the subject to develop the morbidity. While the operation 325 has been described to be performed by the healthcare professional, in some implementations, the computing system, the diagnostic system, or other appropriate system can select the patient for the diagnostic workup.

In cases where the morbidity is anemia, the healthcare professional can determine an optimization of erythropoietin or iron supplementation therapy. For example, the computing system can determine the normalized critical volume $v_c$ or other parameter estimated from a blood sample from a subject undergoing erythropoietin treatment. Based on the normalized critical volume, the computing system can adjust a dose of EPO or iron supplementation to maintain the normalized critical volume ($v_c$) or other parameter at a desired level, e.g., a level above or below a threshold level, or a level within a desired or normal range. For example, as noted herein the normalized critical volume ($v_c$) in normal healthy individuals is about 0.8 of the population mean volume, v, or about 72 fL for a typical MCV of 90 fL. Thus, in some implementations, the computing system may include an operation to adjust the dose of EPO or iron supplementation to maintain the normalized critical volume ($v_c$) to be greater than 0.7 of v in the subject; greater than 0.75 of v in the subject; or between 75-85% of v in the subject.

### Samples, Systems, Software, and Assay Methods and Systems

The methods and systems described herein are typically practiced using peripheral blood samples obtained using known collection methodology that typically preserves RBCs intact (e.g., a blood draw with an appropriate amount of vacuum (draw) and a needle large enough to allow the RBCs to be collected without substantial hemolysis, e.g., a needle of at least 25 g or larger). The measurements are preferably made within 24, 12, or 6 hours of collection. Reticulocyte and CBC measurements can be made using any methods or devices known in the art that can measure both RBC volume (e.g., using low angle (2°-3°) scatter detection) and hemoglobin mass or concentration (e.g., using high angle (5°-15°) scatter detection). Exemplary methods are described in U.S. Published Application Nos. 20110178716, 20110164803, 20110149061, 20110077871, 20110070606, and 20110070210.

The blood sample can be used with a hematology analyzer, an immunoassay analyzer, a hemoglobin testing system, and other appropriate blood testing apparatuses and devices. In some implementations, a hemanalyzer or hematology analyzer (e.g., the hematology analyzer 105 of Fig. 1) measures characteristics of the blood sample. The hemanalyzer can be, for example, a manual, semi-automated, or automated hematology analyzer. Examples of hemanalyzers are described in U.S. Patent Nos. 5,017,497, 5,266,269, 5,378,633, 5,631,165, 5,812,419, 6,228,652, 6,524,858, 6,320,656, 7,324,194, and 7,981,681, as well as published U.S. Patent Application Nos. 20080153170, 20080158561, 20080268494, 20110178716, 20110077871, and 20110070606.

Hemanalyzers useful in the present methods can use any appropriate detection method known in the art, e.g., flow cytometry or optical or image-based analysis or impedance based. Hemanalyzers useful in the present methods and systems can measure the parameters of the CBC described herein, such as, for example, the RBC cell volume, and at least one of the cell hemoglobin concentration and cell hemoglobin mass.

A number of models of hematology analyzers are commercially available, e.g., from Abbott Laboratories (Abbott Park, IL, United States) (e.g., the Cell-Dyn Sapphire); and Siemens (Deerfield, IL, United States) (e.g., the Advia 120 or 2120 automated hemanalyzer). Other manufacturers include Beckman Coulter, Inc. (Fullerton, CA, United States); TOA Medical Electronics Co., (Kobe, Japan); Constitution Medical (Boston, MA); and HORIBA ABX Inc. (Irvine, CA, United States).

The present methods and systems may include hematology analyzer systems. The hematology analyzer systems can include a detection module for measuring a clinical sample (e.g., a blood sample) and a computing device that is in communication with the detection module and comprises programming for determining, based on the output of the detection module, one or more of: (i) a population mean corpuscular hemoglobin concentration (MCHC) and the fraction of red blood cells in the sample whose transformed volume and hemoglobin content fall below a threshold percentage of the average of all transformed volume and hemoglobin contents, to determine a sample fraction; (ii) a magnitude of variation among cells in the population in the rate of hemoglobin content reduction ($D_h$); (iii) a magnitude of variation among cells in the population in the rate of cell volume reduction ($D_v$); and/or (iv) a normalized critical volume ($v_c$) or other parameter indicative of RBC clearance delay. The detection module can include an analysis chamber configured to hold a sample comprising red blood cells, e.g. whole blood, for analysis. In some implementations, the detection module comprises a flow cytometer configured to analyze the sample. In some implementations, the detection module comprises an optical or image analyzer configured to analyze the sample.

Examples of appropriate commercially available immunoassay analyzers (e.g., the immunoassay analyzer 120) can include a Roche COBAS instrument (Roche Diagnostics, Indianapolis, Indiana). Examples of appropriate commercially available hemoglobin testing systems (e.g., the hemoglobin testing system 115) can include a BIO-RAD Variant II Turbo instrument (BIO-RAD, Hercules, California).

In some implementations, a computer-readable medium can include instructions to execute the process 300, including each of the operations 305, 310, 315, 320, and 325 described herein. A computer-readable medium can include instructions to calculate one or more of: (i) a population mean corpuscular hemoglobin concentration (MCHC) and the fraction of RBCs in the sample whose transformed volume and hemoglobin content fall below a threshold percentage of the average of all transformed volume and hemoglobin contents, to determine a sample fraction; (ii) a magnitude of variation among cells in the population in the rate of hemoglobin content reduction ($D_h$); (iii) a magnitude of variation among cells in the population in the rate of cell volume reduction ($D_v$); (iv) a normalized critical volume ($v_c$) or other indicator of RBC clearance delay; and/or (v) other parameters and measurements described herein. See also WO2012/037524.

In some implementations, data from a hematology analyzer, an immunoassay analyzer, and/or a hemoglobin testing system are received (e.g., red blood cell (RBC) cell volume (CV), mean cell volume (MCV), cell hemoglobin concentration (CHC) or cell hemoglobin content (CH), mean cell hemoglobin concentration (MCHC), the mean cell hemoglobin content (MCH), other population statistics, glycated hemoglobin content, ferritin levels, and other measurements of the population of RBCs), by a computing device that then executes instructions to calculate of one or more of (i) a population mean corpuscular hemoglobin concentration (MCHC) and the fraction of red blood cells in the sample whose transformed volume and hemoglobin content fall below a threshold percentage of the average of all transformed volume and hemoglobin contents, to determine a sample fraction; (ii) a magnitude of variation among cells in the population in the rate of hemoglobin content reduction ($D_h$); (iii) a magnitude of variation among cells in the population in the rate of cell volume reduction ($D_v$); and/or (iv) a normalized critical volume ($v_c$) or other indicator of RBC clearance delay.

In some implementations, the computing device is a separate computer, using inputs from the hemanalyzer, the immunoassay analyzer, and/or the hemoglobin testing system. In some implementations, the computing device is integrated into, or part of, the hemanalyzer, the immunoassay analyzer, and/or the hemoglobin testing system. As a result, a single system can include each of the computing device, the hemanalyzer, the immunoassay analyzer, and/or the hemoglobin testing system.

The programming can be provided in a physical storage or transmission medium. A computing device (e.g., a separate device or an information processing module that is part of a hemanalyzer system) receiving the instructions can then execute the algorithm and/or process data obtained from the subject method. Examples of storage media that are computer-readable include floppy disks, magnetic tape, CD-ROM, a hard disk drive, a ROM or integrated circuit, a magneto-optical disk, or a computer readable card such as a PCMCIA card and the like, whether or not such devices are internal or external to the computer. A file containing information can be "stored" on computer readable medium, where "storing" means recording information such that it is accessible and retrievable at a later date by a computer on a local or remote network. In some implementations, the methods described herein are automatically executed each time a sample is run.

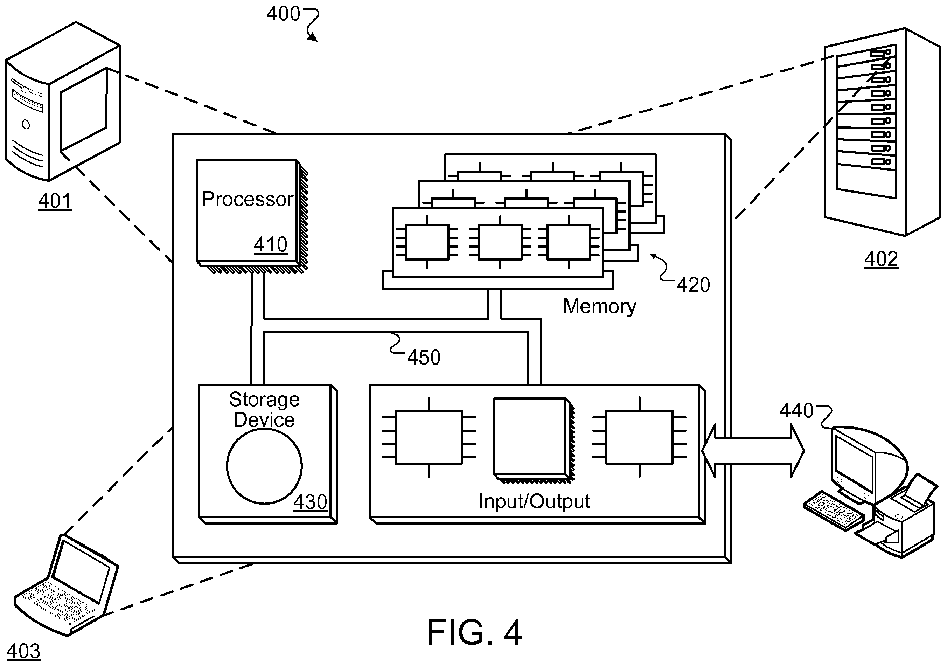

Fig. 4 is a schematic diagram of an example computer system 400 that can be used for operations (e.g., the operations 305, 310, 315, 320, 325 of the process 300 described with respect to Fig. 3) described in association with any of the computer-implemented methods described herein, according to one implementation. The computing system 400 can receive data from external measurement and testing systems (e.g., the hematology analyzer 105, the hemoglobin testing system 115, and the immunoassay analyzer 120), store the data, and process the data. The computing system 400 can further make determinations about a treatment or diagnosis plan for a subject associated with the data.

The system 400 is intended to represent various forms of digital computers, including, e.g., laptops, desktops, workstations, personal digital assistants, servers, blade servers, mainframes, and other appropriate computers, including computers that are incorporated into hemanalyzer systems or devices. The system 400 can be incorporated in various computing devices such as a desktop computer 401, server 402, and/or a laptop computer 403. The system 400 includes a processor 410, a memory storage element 420, a storage device 430, and an input/output device 440. Each of the components 410, 420, 430, and 440 are interconnected using a system bus 450. The processor 410 is capable of processing instructions for execution within the system 400. In one implementation, the processor 410 is a single-threaded processor. In another implementation, the processor 410 is a multi-threaded processor. The processor 410 is capable of processing instructions stored in the memory storage element 420 or on the storage device 430 to display graphical information for a user interface on the input/output device 440.

The memory storage element 420 stores information within the system 400. In some implementations, the memory storage element 420 is a computer-readable medium. The memory storage element 420 can include volatile memory and/or non-volatile memory.

The storage device 430 is capable of providing mass storage for the system 400. In one implementation, the storage device 430 is a computer-readable medium. In various different implementations, the storage device 430 may be a floppy disk device, a hard disk device, an optical disk device, or a tape device.

The input/output device 440 provides input/output operations for the system 400. In some implementations, the input/output device 440 includes a keyboard and/or pointing device. In some implementations, the input/output device 440 includes a display unit for displaying graphical user interfaces. In some implementations the input/output device can be configured to accept verbal (e.g. spoken) inputs. For example, the clinician can provide the input by speaking into the input device.

The features described can be implemented in digital electronic circuitry, or in computer hardware, firmware, or in combinations of these. The features can be implemented in a computer program product tangibly embodied in an information carrier, e.g., in a machine-readable storage device, for execution by a programmable processor; and features can be performed by a programmable processor executing a program of instructions to perform functions of the described implementations by operating on input data and generating output. The described features can be implemented in one or more computer programs that are executable on a programmable system including at least one programmable processor coupled to receive data and instructions from, and to transmit data and instructions to, a data storage system, at least one input device, and at least one output device. A computer program includes a set of instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

Suitable processors for the execution of a program of instructions include, by way of example, both general and special purpose microprocessors, and the sole processor or one of multiple processors of any kind of computer. Generally, a processor will receive instructions and data from a read-only memory or a random access memory or both. Computers include a processor for executing instructions and one or more memories for storing instructions and data. Generally, a computer will also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory can be supplemented by, or incorporated in, ASICs (application-specific integrated circuits).

To provide for interaction with a user, the features can be implemented on a computer having a display device such as a CRT (cathode ray tube) or LCD (liquid crystal display) monitor for displaying information to the user and a keyboard and a pointing device such as a mouse or a trackball by which the user can provide input to the computer.

The features can be implemented in a computer system that includes a back-end component, such as a data server, or that includes a middleware component, such as an application server or an Internet server, or that includes a front-end component, such as a client computer having a graphical user interface or an Internet browser, or any combination of them. The components of the system can be connected by any form or medium of digital data communication such as a communication network. Examples of communication networks include, e.g., a LAN, a WAN, and the computers and networks forming the Internet.

The computer system can include clients and servers. A client and server are generally remote from each other and typically interact through a network, such as the described one. The relationship of client and server arises by virtue of computer programs running on the respective computers and having a client-server relationship to each other.

The processor 410 carries out instructions related to a computer program. The processor 410 may include hardware such as logic gates, adders, multipliers and counters. The processor 410 may further include a separate arithmetic logic unit (ALU) that performs arithmetic and logical operations.

## EXAMPLES

The invention is further described in the following examples, which do not limit the scope of the invention described in the claims.

Examples herein utilize subsets of data collected using the methods described below. An Abbott Cell-DYN Sapphire 4000 automated hematology analyzer (Abbott Hematology, Santa Clara, California) was used to measure CBCs and reticulocyte counts for more than 60,000 randomly selected blood samples from a clinical laboratory. All human studies were approved by the Partners Healthcare Institutional Review Board. For each CBC and reticulocyte count, the hematology analyzer measured the volume and hemoglobin concentration. Both volume and hemoglobin concentration were measured for each individual's RBCs, yielding the joint volume-hemoglobin distribution for the entire population of the blood samples. These measurements were processed for the simulations, modeling, and other data processing described in the examples herein. Measurements were also taken on the Abbott Cell-DYN Sapphire 4000 as well as Siemens Advia 120, 2120, and 2120i instruments to validate the measurements for the simulations, modeling, and data processing described herein. All CBC parameters used in hypothesis testing were measured on instruments including the Siemens Advia 2120 and the Sysmex XE-5000.

Other testing was performed on instruments configured to measure, for example, ferritin levels and glycated hemoglobin content. CBC and reticulocyte counts for each blood sample were modeled as described herein. Blood samples with appropriate CBC parameters and normalized critical volumes ($v_c$) were then selected for further measurement of ferritin and hemoglobin A1c. Ferritin levels were measured on a Roche COBAS instrument (Roche Diagnostics, Indianapolis, Indiana). Glycated hemoglobin was measured on a BIO-RAD Variant II Turbo instrument (BIO-RAD, Hercules, California).

*Example 1: Measurements and Modeling*

A hematology analyzer was used to measure 600 blood samples from a population of healthy patients. Hemoglobin content (g/dl), RDW (%), hematocrit (%), and rRDW (%), among other characteristics, were measured for each of the blood samples. Healthy patients met the following criteria:

(1) The patient had at least three CBCs spanning at least 3 years.
(2) The patient had no recorded abnormal CBC index. See Table 1 herein for examples of normal ranges.

From randomly selected medical records from the population of healthy patients, a prevalence of chronic illness was determined to be below 5%. The characteristics measured from the healthy population were used to estimate lower bounds on the typical variation of clinical parameters in a truly healthy population. The population of healthy patients was 43% male, with a median age of 53 years and an interquartile range of 23 years. There was no overlap between this patient cohort and any of the other patient cohorts in other examples described herein.

The measurements from the hematology analyzer served as inputs for a mathematical model of *in vivo* RBC population dynamics (See, e.g., Reference 9). The mathematical model inferred single-RBC rates of maturation and clearance for each of the blood samples from individual patients. All data analysis and modeling was done in MATLAB (MathWorks, Natick, Massachusetts).

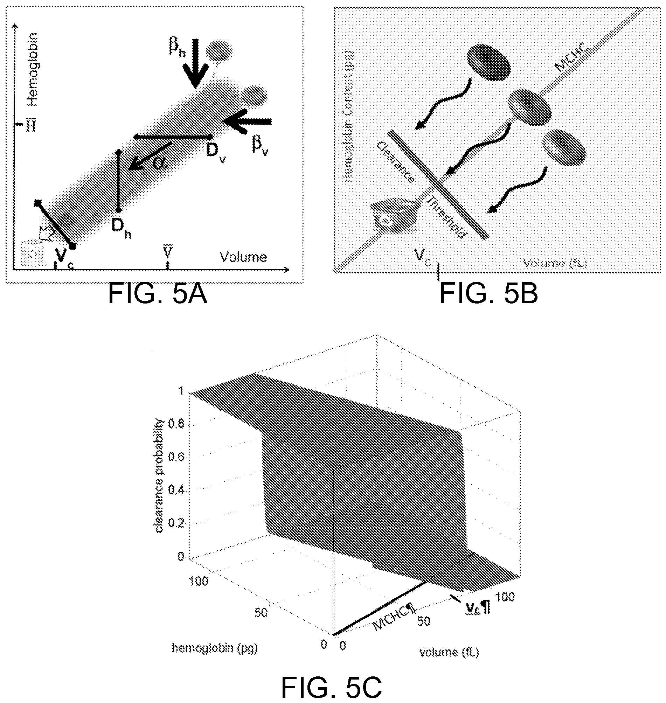

The mathematical model quantified parameters characterizing the population dynamics of each of the population of RBCs of each of the blood samples. Fig. 5A shows a graph depicting the parameters quantified by the mathematical model. The x-axis represents the volume, and the y-axis represents the hemoglobin content of individual cells or means of the population of the RBCs. Single-RBC measurements of volume and hemoglobin content were used to estimate the rates of maturation and clearance. RBCs were assumed to become smaller and lose hemoglobin as they age, with an initial fast phase of reduction followed by a slow phase (See, e.g., References 10, 13, and 15). As shown in Fig. 5A, the model estimated each of a magnitude of variation in a rate of hemoglobin content reduction ($D_h$), a magnitude of variation in a rate of RBC volume reduction ($D_v$), a normalized clearance volume ($v_c$) indicative of a volume in which clearance occurs for most of the population of RBCs, an average rate of slow-phase RBC volume and hemoglobin content reduction ($\alpha$), an average rate of fast-phase RBC volume reduction ($\beta_v$), and an average rate of fast-phase RBC hemoglobin content reduction ($\beta_h$). The model quantified the magnitude of variation in rates of both hemoglobin ($D_h$) and volume reduction ($D_v$). The model quantified the rates of the initial fast phase for each individual by model parameters $\beta_v$ and $\beta_h$. The model quantified the slower rate with the parameter $\alpha$. Rates of volume and hemoglobin reduction varied for a single RBC over time and from one RBC to the next in the population.

The model approximated the RBC clearance as a threshold function of RBC volume and hemoglobin. The threshold function was defined by the critical volume or the normalized clearance volume ($v_c$) shown in Fig. 5B. The model showed that the probability of RBC clearance were correlated with an RBC's volume and hemoglobin, and the true clearance function was approximated as a threshold function of RBC volume and hemoglobin. The function thus approximated the probability of clearance for a particular RBC to increase rapidly as the cell nears a threshold line defined by the normalized critical volume ($v_c$). The threshold line is perpendicular to and intersects the MCHC (mean corpuscular hemoglobin concentration) line at a volume equal to the normalized critical volume ($v_c$). The model determined an MCHC line from the MCHC and then computed a probability of clearance of the RBCs of the population of the RBCs based on the position of the normalized critical volume ($v_c$) relative to the MCHC line.

As shown in Fig. 5C, the probability of clearance as a function of RBC volume and hemoglobin in the model increases rapidly from 0% to 100%. The 3D graph of Fig. 5C shows the effects of each of the volume and the hemoglobin content of the RBCs on the clearance probability. The threshold function determined by the normalized critical volume ($v_c$) is depicted as the threshold line perpendicular to the MCHC line such that, if the projected volume and hemoglobin content of the RBC is below the threshold line, the probability of clearance is 100%, and if the projected volume and hemoglobin content of the RBC is above the threshold line, the probability of clearance is 0%. An RBC clearance delay would be indicated as a shift toward the origin of the surface of Fig. 5C representing the probability distribution of RBC clearance.

The model simulated that an RBC with a hemoglobin concentration equal to the MCHC was most likely to be recycled when its volume reached $v_c$. The population dynamics approximated by the model further simulated a mechanism in which RBCs with higher hemoglobin concentrations would circulate until reaching slightly lower volumes, and RBCs with lower hemoglobin concentrations would be cleared at slightly higher volumes. Examples of such models of population dynamics are described in Reference 9 listed herein.

The model, defined by Equation 1 below, described the volume and hemoglobin dynamics of a typical RBC $\left(\frac{dv}{dt}, \frac{dh}{dt}\right)$ as a function ($f$) of its current volume and hemoglobin and accounting for fluctuations in these rates of volume and hemoglobin loss over time ($\zeta$). The dynamics of volume and hemoglobin in the full RBC population $\left(\frac{dP}{dt}\right)$ included production of reticulocytes (b) and clearance of senescent cells (c).

$$\text{Equation (1): } \begin{bmatrix} dv \\ dh \end{bmatrix} = \boldsymbol{f}\, dt + d\boldsymbol{\xi}(t)$$

Each of the elements of Equation (1) were characterized as follows:

$$f = \begin{Bmatrix} -\alpha \cdot e^{\beta_v(v-h)} \\ -\alpha \cdot e^{\beta_h(h-v)} \end{Bmatrix}$$

$$\xi(t) = \begin{Bmatrix} \sqrt{2D_v} \cdot B^1(t) \\ \sqrt{2D_h} \cdot B^2(t) \end{Bmatrix}$$

where $B^1(t)$ are independent Brownian motion.

$$\frac{dP(v,h,t)}{dt} = -\nabla(P\boldsymbol{f}) + \nabla(\boldsymbol{D}\nabla P) + b(v,h) - c(v,h) \cdot P$$

where $b(v, h)$ is the scaled empirical reticulocyte distribution.

$$c(v,h) = \frac{1}{1 + e^{\Delta(v,h)}}$$

$$\Delta(v,h) = 100\frac{\cos(\theta)\sqrt{v^2 + h^2} - v_c\sqrt{\bar{v}^2 + \bar{h}^2}}{v_c\sqrt{\bar{v}^2 + \bar{h}^2}}$$

$$\theta = tan^{-1}\left(\frac{\bar{h}}{\bar{v}}\right) - tan^{-1}\left(\frac{h}{v}\right)$$

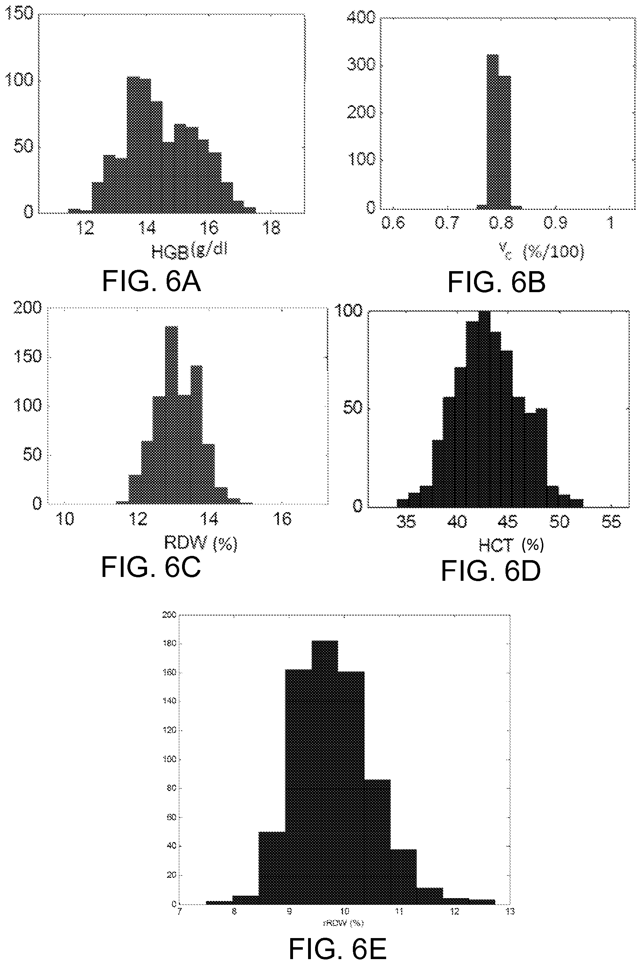

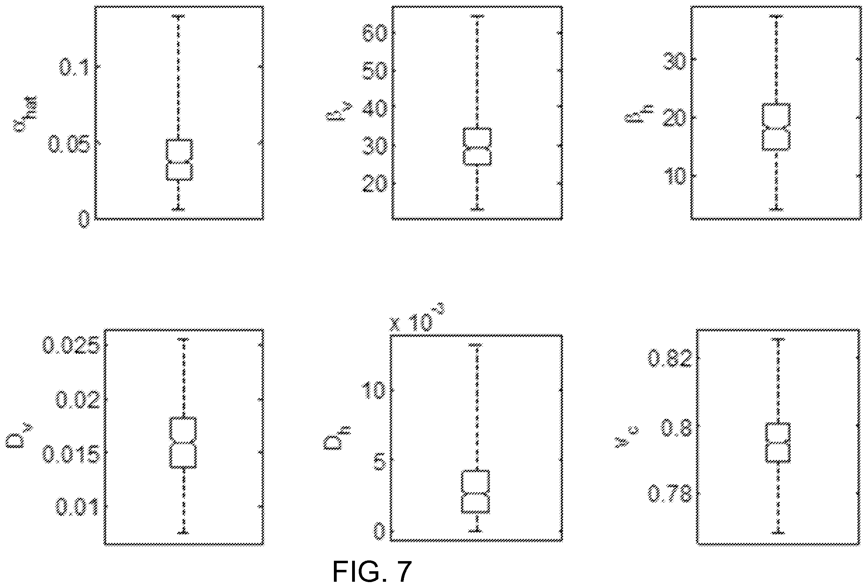

Figs. 6A to 6E show histograms of parameters and measurements related to the population of the blood samples (n = 600). From the measurements of the hematology analyzer, a distribution of hemoglobin (as shown in Fig. 6A), a distribution of RDW (as shown in Fig. 6C), a distribution of hematocrit (as shown in Fig. 6D), and a distribution of rRDW (as shown in Fig. 6E) in the population of the blood samples were determined. The model determined $v_c$ for each of the blood samples, and then a distribution of $v_c$ (as shown in Fig. 6B) was determined. The model and measurements thus showed that $v_c$ was more tightly regulated in a healthy population, having a coefficient of variation of 1.1%. The coefficient of variation for the hemoglobin distribution was 7.9%. The coefficient of variation for the RDW distribution was 4.5%. The coefficient of variation for the hematocrit distribution was 7.5%. The coefficient of variation in rRDW distribution was 8.2%. The model further determined the distribution of each of the estimated parameters, including $D_h$, $D_v$, $v_c$, $\alpha$, $\beta_v$, and $\beta_h$, in the population of the blood samples, as shown in Fig. 7. For $D_h$, the mean was 0.00283, the median was 0.00243, the 25th percentile was 0.00134, the 75th percentile was 0.00414, the minimum value was le-15, and the maximum value was 0.141. For $D_v$, the mean was 0.0159, the median was 0.0160, the 25th percentile was 0.0136, the 75th percentile was 0.0186, the minimum value was 0.00505, and the maximum value was 0.0256. For $v_c$, the mean was 0.795, the median was 0.795, the 25th percentile was 0.789, the 75th percentile was 0.801, the minimum value was 0.753, and the maximum value was 0.826. For $\alpha$, the mean was 0.0424, the median was 0.0390, the 25th percentile was 0.0257, the 75th percentile was 0.0520, the minimum value was 0.00606, and the maximum value was 0.133. For $\beta_v$, the mean was 29.2, the median was 28.5, the 25th percentile was 24.9, the 75th percentile was 34.5, the minimum value was 12.1, and the maximum value was 64.3. For $\beta_h$, the mean was 17.9, the median was 17.6, the 25th percentile was 14.6, the 75th percentile was 22.4, the minimum value was 1.43, and the maximum value was 37.4. The coefficients of variation of each of the parameters and measurements described in this example demonstrate that the RBC clearance delay is more correlated with the estimated $v_c$ than other parameters and measurements.

While Example 1 describes a specific example of simulating the population dynamics of the population of RBCs, other implementations can vary. For example, instead of a threshold function to represent the probability of clearance, the model can include a sigmoid function, a step function, a polynomial function, a logarithmic function, or other appropriate function to simulate the probability of clearance of RBCs.

*Example 2: Correlation between RBC Clearance Delay and Elevated RDW*

To assess the correlation between the possible causes of elevated RDW across a general inpatient and outpatient hospital patient population, 450 blood samples were selected from the clinical laboratory and were processed through the dynamic population simulations as described herein. This cohort was 45% male, with a median age of 59 years and an interquartile range of 26 years. There was no overlap between this patient cohort and any of the other patient cohorts used in other examples described herein.

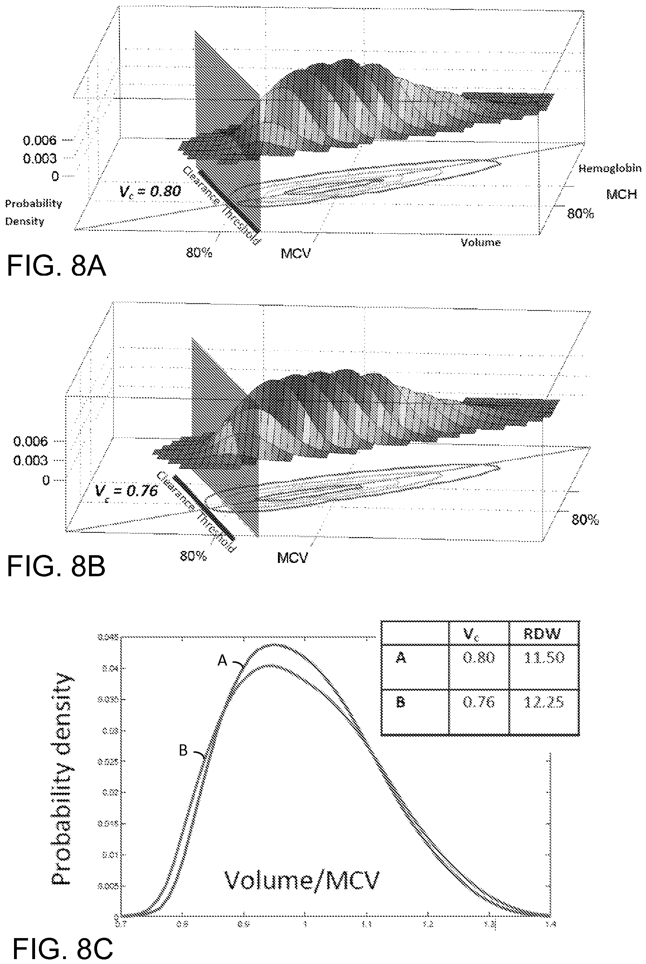

The model of RBC population dynamics described in Example 1 herein was used to simulate an effect of delaying RBC clearance by lowering $v_c$ from 0.8 to 0.76. Volume and hemoglobin are scaled by their means in Figs. 8A to 8C. Fig. 8A shows the probability density for clearance of an RBC based on volume and hemoglobin of the RBC for a healthy individual with an estimated normalized clearance volume ($v_c$) of 0.80. The model executed the simulation of population dynamics described herein to estimate the normalized clearance volume ($v_c$). Using the model generated from Fig. 8A, an RBC clearance delay of 5% (e.g., decreasing $v_c$ from 0.8 to 0.76) was simulated.

Fig. 8B depicts that the RDW increased as a result of the RBC clearance delay of 5%. The contours shown in Figs. 8A and 8B enclose 95%, 75%, 50%, and 25% of RBCs. The center of the contours is the mean cell volume of the population of RBCs. Because delayed RBC clearance enables RBCs to continue circulating with smaller volumes than would be possible otherwise, the low-volume tail of the RBC volume distribution is expanded and extended. As shown in Fig. 8B, the low-volume tail expanded beyond the original $v_c$ marked with a plane at 0.80. As shown in Fig. 8C, the decrease in $v_c$ raised the RDW from 11.5% to 12.25%. Fig. 8C further shows the concurrent effect that the low-volume tail of graph B (corresponding to the simulated effects of decreasing the normalized clearance volume $v_c$ to 0.76) is expanded relative to graph A, thus suggesting the correlation between $v_c$ and RDW.

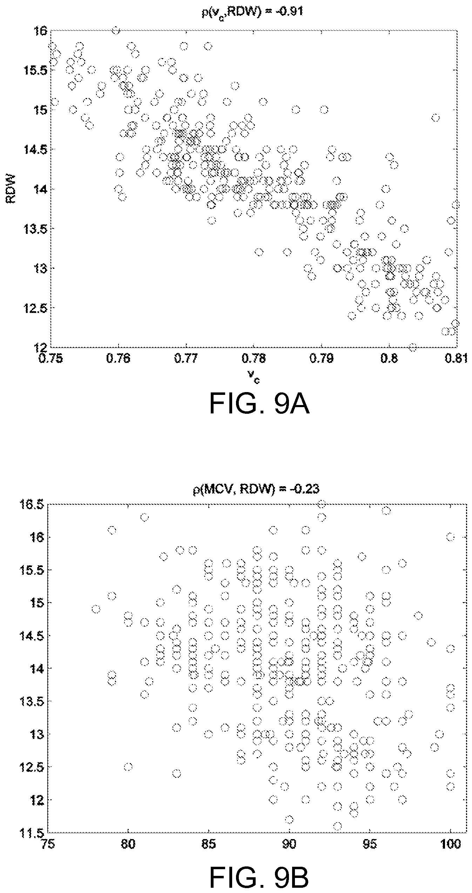

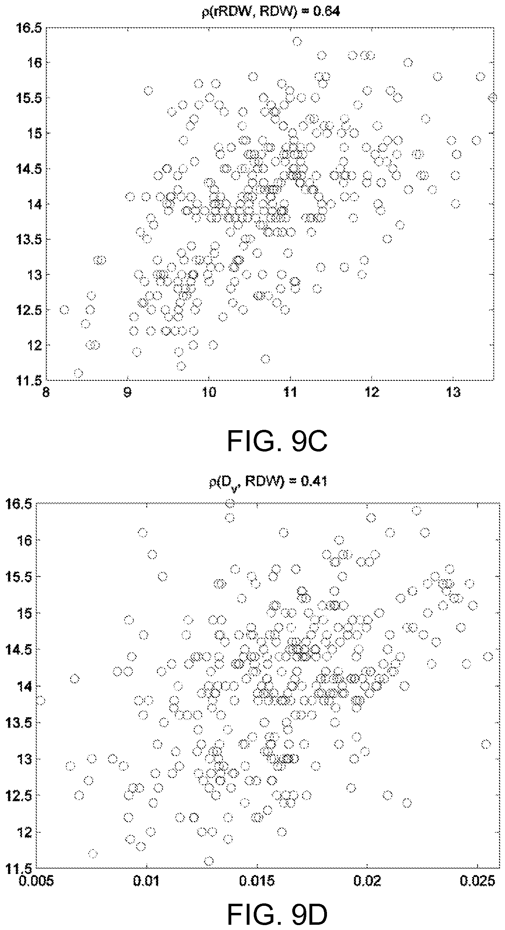

Figs. 9A to 9D each show parameters measured in each of the 450 samples measured against RDW. As shown in Figs. 9A to 9D, each of $v_c$, MCW, rRDW, and $D_V$ were measured against RDW to determine a correlation between the parameters and measurements with RDW. As shown in Fig. 9A, $v_c$ strongly correlated with RDW. Figs. 9B to 9D show that MCW, rRDW, and $D_V$ do not as strongly correlate with RDW. As shown in Fig, 9A, the correlation coefficient between $v_c$ and RDW is -0.91 in the randomly selected population of 450 blood samples. The correlation between RDW and $v_c$ is much stronger than the correlation between RDW and any of MCV, rRDW, and $D_V$. MCV had a correlation coefficient of -0.23, as shown in Fig. 9B; rRDW had a correlation coefficient of 0.64, as shown in Fig. 9C; and $D_V$ had a correlation coefficient of 0.41, as shown in Fig. 9D. The normalized clearance value $v_c$ was thus shown to be strongly correlated with RDW.

*Example 3: Association between RBC Clearance Delay, Elevated RDW, and Increased Mean RBC Age*

The model enabled the determination of the magnitude of the clearance delay required to generate the ~0.5% increase in RDW shown to be broadly associated with morbidity and mortality (See, e.g., References 3 and 19). Using, for example, the 450 samples taken as part of Example 2, the $v_c$ and estimated mean RBC age can be determined. The $v_c$ for a particular sample can then be adjusted, and the new mean RBC age be calculated using the model. The model showed that a delay in clearance of less than 3%, for instance, a drop in $v_c$ from 0.79 to 0.77, increased the RDW by ~0.5% (e.g., from 13.2% to 13.7%) and the mean RBC age by 3 to 7% (e.g., from 50 to 51.5-53.5 days). The example described below sought to test this model-generated prediction of increased mean RBC age by estimating mean RBC age and $v_c$.

Elevated RDW was shown to be associated with elevated mean RBC age by computing $v_c$ and the glycated hemoglobin fraction in non-diabetics. This example estimated the RBC clearance rate by calculating $v_c$ using the model as described herein. Because an RBC clearance delay increases RDW and enables cells to circulate beyond the time when they would ordinarily have been cleared, average RBC age and lifespan increases as well as the fraction of the oldest cells. This example examined the relationship between $v_c$ and glycated hemoglobin fraction from 189 blood samples from non-diabetic individuals to confirm and validate the relationship between $v_c$ and the RBC clearance rate.

The glycated hemoglobin fraction (hemoglobin A1c or HbAlc) was measured in non-diabetic individuals to estimate mean RBC age. HbAlc is proportional to the mean plasma glucose concentration and to the mean RBC age (See, e.g., References 20 to 22). In non-diabetic individuals, HbAlc is linearly dependent on mean RBC age (See, e.g., References 14, 20, and 23 to 27). Non-diabetic individuals were those who met the following criteria:

(1) The patient had normal non-fasting glucose at the time of the complete blood count.
(2) The patient's medical record did not have a diagnosis of diabetes.
(3) The patient's medical record did not have any recorded abnormally high glucose measurements within the two years prior to the CBC.
(4) The patient's medical record did not have hemoglobin A1c measurements outside reference ranges described herein.

There was no overlap between this patient cohort and any of the other patient cohorts used in other examples described herein. The patients in this cohort were 47% male and had a median age of 52 years with an interquartile range of 30 years. Table 2 shows examples of normal ranges.

Blood samples from 74 of the non-diabetic individuals were determined to have low normalized critical volumes, defined to be a $v_c$ less than 0.775. These low $v_c$ group had a median $v_c$ of 0.77. Blood samples from 115 of the non-diabetic individuals were determined to have high normalized critical volumes, defined to be a $v_c$ greater than 0.79. The high $v_c$ group had a median $v_c$ of 0.8. The low $v_c$ group thus had a delay in RBC clearance as compared to the normal $v_c$ group.

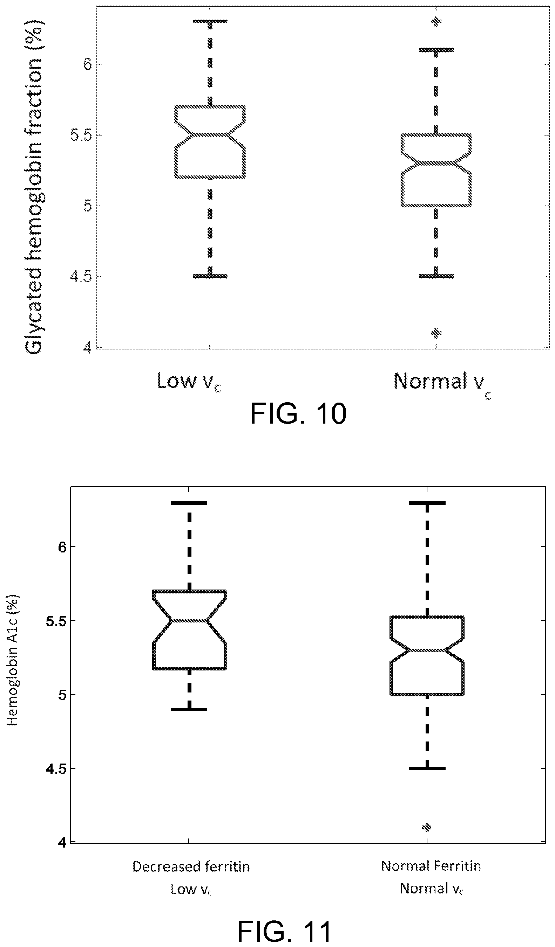

The glycated hemoglobin fraction for a non-diabetic group with normal $v_c$ (median 0.80) was compared to the glycated hemoglobin fraction of a non-diabetic group with low $v_c$ (median 0.77). Fig. 10 shows boxplots of the glycated hemoglobin fractions for 74 non-diabetic individuals with $v_c < 0.775$ (median $v_c = 0.77$) and 115 individuals with $v_c > 0.79$ (median $v_c = 0.80$). The median of the low $v_c$ group had a glycated hemoglobin fraction of 5.5%, and the median of the normal $v_c$ group had a glycated hemoglobin fraction of 5.3%. Based on published estimates of *in vivo* glycation rates in non-diabetics, an approximate 3 to 7% increase in RBC lifespan was expected to increase the glycated hemoglobin fraction by about 0.2% to 0.3% (e.g., from 5.3% in the normal $v_c$ group to about 5.5% or 5.6% in the low $v_c$ group) (See, e.g., Reference 20); as a result, the low $v_c$ group had an estimated 5-day increase in average RBC age compared to the normal $v_c$ group. The medians were evaluated using the Kruskall-Wallis test with p < 0.002. The low $v_c$ group had a median RDW of 14.6%, and the high $v_c$ group had a median RDW of 12.9%. The notches show the 95% confidence intervals for the medians, (5.41, 5.59) is the confidence interval for the median in the low $v_c$ group and (5.23, 5.37) in the normal group. The boxes extend from the 25th to the 75th percentile for each group (5.2, 5.7) is the inter-quartile range for the low $v_c$ group and (5.0, 5.5) is the inter-quartile range for the normal.

The results depicted in Fig. 10 showed that $v_c$ and increased RDW in non-diabetics was associated with elevated glycated hemoglobin fractions and thus increased mean RBC age. In conjunction with the correlation between $v_c$ and the RBC clearance delay established in Example 2 (see, e.g., Fig. 9A), the association between the increased mean RBC age and $v_c$ further supports the conclusion that delayed RBC clearance is the predominant cause of elevated RDW. At least two recent independent studies of 15,343 non-diabetics in the United States and 4,845 non-diabetics in Sweden found an association between increased RDW and increased glycated hemoglobin fraction with high statistical significance (See, e.g., References 28 and 29). Example 3 thus showed that $v_c$, which served as an estimate of the RBC clearance rate, can predict an occurrence of elevated RDW and thus morbidities associated with elevated RDW.

*Example 4: Association between RBC Clearance Delay and Increased Odds of All-cause Mortality*

This example showed that $v_c$ was as predictive of all-cause mortality as elevated RDW. CBCs and reticulocyte counts were measured for patients randomly selected from among those who had complete blood counts as part of their medical care between June, 2012 and December 2013. 900 of those patients ("cases") died by June 2014 according to the Social Security Death Master File. For each case, another patient ("controls") was selected who

(1) had the same gender as the case,
(2) had a date of birth within one year of the case,
(3) had a CBC and reticulocyte measurement within 30 days of the case, and
(4) was not reported as deceased in the Social Security Death Master File.

Some potential biases were controlled in patient selection by selecting cases and controls from the same cohort and by matching them for age, gender, and timing of the CBC used for the comparison. There was no overlap between this patient cohort and any of the other patient cohorts used in examples described herein. The patients in this cohort were 50% female and had a median age of 71 years with an interquartile range of 26 years.

From the 900 cases described above, a logistic regression model was fit to the data to determine an odds of death as a function of the patient's $v_c$. Examples of the odds of death determined as part of this example are shown in Table 5 below:

Table 5: Odds of All-Cause Mortality as a Function of $v_c$ and RDW

| log(odds of death) = Constant + $\beta_1$•Age + $\beta_2$•$v_c$ | | | |
|---|---|---|---|
| | Coefficient | Standard Error | p-value |
| Constant | 23.0 | 4.1 | $< 10^{-5}$ |
| Age | 0.003 | 0.008 | 0.7 |

(continued)

| log(odds of death) = Constant + $\beta_1$•Age + $\beta_2$•$v_c$ | | | | |
|---|---|---|---|---|
| | Coefficient | Standard Error | p-value | |
| $v_c$ | -30.0 | 5.3 | < 10$^{-5}$ | |

| log(odds of death) = Constant + $\beta_3$•Age + $\beta_4$•RDW | | | | |
|---|---|---|---|---|
| | Coefficient | Standard Error | p-value | |
| Constant | -8.3 | 1.4 | < 10$^{-5}$ | |
| Age | 0.002 | 0.008 | 0.8 | |
| RDW | 0.55 | 0.09 | < 10$^{-5}$ | |

A decrease in $v_c$ of 0.01 (approximately 1 standard deviation), for instance from 0.79 to 0.78, increased the odds of death by about $e^{0.23}$= 1.26 or 26% (p-value < 10$^{-10}$). An increase of 0.6 in RDW (approximately 1 standard deviation) in RDW, for instance from 12.5% to 13.1%, increased the odds of death by about the same 26% in a model including age and RDW as predictors (p-value < 10$^{-5}$). The logistic regression coefficients for $v_c$ and RDW had a correlation coefficient of -0.99. This example thus showed that reductions in $v_c$ resulted in both an elevation in RDW as well as an increase in the risk of all-cause mortality within, for example, 2 years (e.g., from June 2012 to June 2014).

*Example 5: Association between RBC Clearance Delay and Increased Odds of Future RDW-associated Pathology*

Risk of all-cause anemia is one condition associated with elevated RDW (See, e.g., References 5 and 6). Odds ratio for $v_c$ and subsequent all-cause anemia were determined in this example. A decrease in $v_c$ was shown to precede both the elevation in RDW and the future clinical appearance of RDW-associated pathologic conditions.

Case patients were individuals presenting with mild anemia who had been hematologically stable within the prior 3 months. Mild anemia was defined as an HCT no more than 10% below the lower limit of the HCT reference range, or 33% <= HCT < 36% for females, and 37% <= HCT < 41% for males. Hematologic stability was defined by the following criteria:

(1) No anemic CBC in the prior 6 months
(2) No CBC within the prior 30 days
(3) No transfusion or surgery in the prior 6 months

Control patients were hematologically stable individuals with two completely normal CBCs separated by between 10 and 14 months, where the second measured hematocrit was no less than the first measured hematocrit. There was no overlap between this patient cohort and any of the others. The patients in this cohort were 35% male and had a median age of 54 years with an interquartile range of 30 years. See Table 2 for examples of normal ranges.

Blood samples from 397 patients were evaluated. 299 patients having blood samples with normal CBCs went on to develop anemia in the subsequent 3 months due to any cause. At the time of their normal CBC as many as 3 months prior to developing anemia, 15% of the 299 patients (44/299) had a low $v_c$, defined to be beneath the 5$^{th}$ percentile of the $v_c$ shown in Fig. 6B (0.7798). The remaining 98 patients from the pool of 397 patients evaluated had blood samples with normal CBCs and exhibited no decrease in hematocrit in the subsequent year. Only 3% (3/98) of those 98 patients had a low $v_c$. The odds ratio for the association between low $v_c$ and future anemia is 4.8, with a 95% confidence interval (1.5, 15.8). Thus, $v_c$, which estimated the RBC clearance delay in this example, can be used to predict pathological conditions associated with elevated RDW, such as all-cause anemia.

This example further showed that $v_c$ precedes future anemia with a greater likelihood than existing CBC indices precede future anemia. An example of the odds ratios determined as part of this example is shown herein in Tables 3 and 4. Confidence intervals in Tables 3 and 4 for odds ratios were calculated by assuming a log-normal distribution. The predictive accuracy of varying thresholds of $v_c$ was compared to the predictive accuracy of varying thresholds of existing CBC indices (e.g., HCT, HGB, MCV, RDW, MCH, and MCHC). $v_c$ reached a maximum odds ratio of more than 8.0. Most of the existing CBC indices, including HCT, HGB, MCH, MCHC, and MCV, have no statistically significant odds ratios for any threshold value. RDW has thresholds with significant odds ratios, but the odds ratio (2.0) for the optimal threshold is less than a quarter of the odds ratio for $v_c$ (8.5). $v_c$ thus predicts more accurately than existing CBC indices even for entirely normal CBCs. RBC clearance delay, which is estimated by $v_c$, thus serves as an early response to a wide range of pathologic conditions associated with elevated RDW.

In this example, a delay in RBC clearance may be represented as an adaptation to mild anemia associated with many of the diseases linked to elevated RDW, maintaining circulating red cell mass and oxygen carrying capacity despite reduced erythropoietic output. Studies have shown that increases on the order of 0.5% in RDW (e.g., from 13.2% to 13.7%) are associated with increased morbidity and mortality risk (See, e.g., References 3 and 19). The modeling in this example suggested that this 0.5% increase in RDW can be caused by a delay in RBC clearance that increased mean RBC age by as about 3%, e.g., from a mean age of 50 days to a mean age of 51.5 days. The example further predicted that this magnitude of clearance delay could additionally cause a slight decrease in MCV, a finding also seen with elevated RDW (See, e.g., Reference 3). The ~3% delay in RBC clearance relative to the individual's baseline could be adaptive compensation for a corresponding ~3% decrease in erythropoietic output. A ~3% reduction in erythropoietic output could indicate a ~3% reduction in reticulocyte production relative to baseline, e.g., a reduction in reticulocyte count from 1.00% to 0.97%. In a typical complete blood count of ~50,000 RBCs, the absolute number of reticulocytes would drop from 500 to 485. This ~3% relative drop in reticulocyte count (~15 fewer absolute reticulocytes counted per 50,000 RBCs in a CBC) would be difficult to detect by reticulocyte count assays which show > 3x imprecision (~10%) even on repeat measurements of the same patient blood sample and have coefficients of variation in healthy populations of at least 30% based on the results described herein and those previously published (See, e.g., Reference 30).

*Example 6: Association between RBC Clearance Delay and a Higher Relative Risk for Current Latent Pathology*

This example shows that low $v_c$ can indicate decreasing or decreased iron availability, even in individuals whose hematocrit measured as part of the CBC has not decreased from normal ranges. As a result, an individual may have abnormal levels of iron but may appear non-anemic using standard CBC measurements of, for example, hematocrit. Individuals can become anemic due to iron deficiency. In some cases, iron deficiency anemia is associated with elevated RDW at the time of diagnosis (See, e.g., Reference 6). In some individuals whose hematocrit has not decreased at all, RBC clearance delay may be occurring in response to decreases in erythropoietic output. As a result, RBC clearance delay in patients having normal levels of hematocrit can be indicative of decreased iron stores.

Ferritin levels of individuals with normal CBCs and low $v_c$ were measured and compared to ferritin levels of controls with $v_c$ above the median. All individuals were hematologically stable with completely normal CBCs and a range of $v_c$ were measured. Hematologically stable individuals were defined as those meeting the following criteria:

(1) No anemia in the previous 6 months
(2) No hospital visits in the prior 30 days

Decreased iron stores was defined as ferritin levels in the bottom 5 percent of the normal range or below, equivalent to a ferritin < 20 ng/dl for females (normal range 10-200) and a ferritin < 44 ng/dl for males (normal range 30-300). There was no overlap between this patient cohort and any of the other patient cohorts in the examples described herein. The patients in this cohort were 39% male and had a median age of 59 years with an interquartile range of 26 years.

Individuals with low $v_c$ had a higher prevalence of decreased iron stores as compared to the prevalence of decreased iron stores for individuals with normal $v_c$. Referring to Fig. 10, boxplots show glycated hemoglobin in non-diabetics with normal CBCs and either ferritin below the bottom 5% of the normal range and a low $v_c$ or ferritin above the bottom 5% of the reference range and normal $v_c$. The decreased ferritin and low $v_c$ group had 29 individuals with median HbAlc of 5.5% and median ferritin of 17 ng/ml. The normal ferritin and normal $v_c$ group had 109 individuals with median HbAlc of 5.3% and median ferritin of 107 ng/ml. 10/68 (16%) patients with low $v_c$ and in 4/132 (3%) patients with $v_c$ above the median were determined to have decreased iron stores. Thus, among patients with normal CBCs, and in particular, normal levels of hematocrit, the risk of decreased iron stores for those with low $v_c$ is 4.9 relative to those with normal $v_c$ with a 95% confidence interval of (1.6, 14.7). Calculating $v_c$ may thus help identify patients with delayed RBC clearance who would benefit from further diagnostic workup for diseases and conditions that can underlie iron deficiency such as, for example occult GI bleeding due to undiagnosed colon cancer or insufficient dietary intake during important developmental periods (See, e.g., References 31 and 32).

To confirm that the decreased ferritin levels in the low $v_c$ individuals coincided with an increased RBC lifespan indicative of delayed RBC clearance, the glycated hemoglobin was measured for the same individuals. As described herein, glycated hemoglobin can provide an estimate of mean RBC age in non-diabetics (See, e.g., References 14, 20, 23-27). The measured hemoglobin A1c levels in the non-diabetics among these individuals with decreased ferritin and low $v_c$ were found to be increased relative to the non-diabetics in the group with normal ferritin and $v_c$. Thus, the decreased ferritin was associated with delayed RBC clearance.

The foregoing examples-not intended to be limiting to the scope of the invention-demonstrate that computing $v_c$ enables the estimation of the RBC clearance rate and therefore the identification of cohorts of patients in the latent or incipient stage of many important diseases, while the disease or its complications are still invisible to current clinical methods. In particular, the delayed RBC clearance estimated by $v_c$ can precede elevated RDW in general. In many pathologic conditions, decreased $v_c$ correlates with RDW much more strongly than the other potential causes, (2) individuals with increased RDW have a mild increase in average RBC age that can indicate a relationship between RBC clearance delay and elevated RDW, (3) decreased $v_c$ correlates with all-cause mortality risk just as strongly as RDW, (4) among individuals with normal CBCs a low $v_c$ is associated with a higher odds ratio for future RDW-associated pathology such as, for example anemia, and (5) individuals with low $v_c$ have a higher relative risk for current latent pathology like decreased iron stores.

**REFERENCES**

1. Home BD. A Changing Focus on the Red Cell Distribution Width: Why Does It Predict Mortality and Other Adverse Medical Outcomes? Cardiology 2012;122:213-215.

2. Felker GM, Allen LA, Pocock SJ, et al. Red cell distribution width as a novel prognostic marker in heart failure - Data from the CHARM program and the Duke Databank. J Am Coll Cardiol 2007;50:40-47.

3. Perlstein TS, Weuve J, Pfeffer MA, et al. Red Blood Cell Distribution Width and Mortality Risk in a Community-Based Prospective Cohort. Arch Intern Med 2009;169:588-594.

4. Anderson JL, Ronnow BS, Home BD, et al. Usefulness of a complete blood count-derived risk score to predict incident mortality in patients with suspected cardiovascular disease. Am J Cardiol 2007;99:169-174.

5. Spell DW, Jones DV, Harper WF, et al. The value of a complete blood count in predicting cancer of the colon. Cancer Detect Prev 2004;28:37-42.

6. Pascual-Figal DA, Bonaque JC, Manzano-Fernandez S, et al. Red blood cell distribution width predicts new-onset anemia in heart failure patients. Int J Cardiol 2012; 160:196-200.

7. Allen LA, Felker GM, Mehra MR, et al. Validation and Potential Mechanisms of Red Cell Distribution Width as a Prognostic Marker in Heart Failure. J Card Fail 2010;16:230-238.

8. Home BD. The Red Cell Distribution Width: What Is Its Value for Risk Prognostication and for Understanding Disease Pathophysiology? Cardiology 2011; 119:140-141.

9. Higgins JM, Mahadevan L. Physiological and pathological population dynamics of circulating human red blood cells. Proc Natl Acad Sci U S A 2010;107:20587-20592.

10. Willekens FLA, Werre JM, Groenen-Dop YAM, et al. Erythrocyte vesiculation: a self-protective mechanism? Br J Haematol 2008; 141:549-556.

11. Willekens FL, Roerdinkholder-Stoelwinder B, Groenen-Dopp YA, et al. Hemoglobin loss from erythrocytes in vivo results from spleen-facilitated vesiculation. Blood 2003;101:747-751.

12. Waugh RE, Narla M, Jackson CW, et al. Rheologic Properties of Senescent Erythrocytes - Loss of Surface-Area and Volume with Red-Blood-Cell Age. Blood 1992;79:1351-1358.

13. Franco RS, Puchulu-Campanella ME, Barber LA, et al. Changes in the properties of normal human red blood cells during in vivo aging. Am J Hematol 2013;88:44-51.

14. Franco RS. The measurement and importance of red cell survival. Am J Hematol 2009;84:109-114.

15. Gifford SC, Derganc J, Shevkoplyas SS, et al. A detailed study of time-dependent changes in human red blood cells: from reticulocyte maturation to erythrocyte senescence. Br J Haematol 2006;135:395-404.

16. Willekens FLA, Roerdinkholder-Stoelwinder B, Groenen-Dopp YAM, et al. Hemoglobin loss from erythrocytes in vivo results from spleen-facilitated vesiculation. Blood 2003;101:747-751.

17. Beutler E, Waalen J. The definition of anemia: what is the lower limit of normal of the blood hemoglobin concentration? Blood 2006;107:1747-1750.

18. Donofrio G, Chirillo R, Zini G, et al. Simultaneous Measurement Of Reticulocyte And Red-Blood-Cell Indexes In Healthy-Subjects And Patients With Microcytic And Macrocytic ANEMIA. Blood 1995;85:818-823.

19. Patel KV, Ferrucci L, Ershler WB, et al. Red Blood Cell Distribution Width and the Risk of Death in Middle-aged and Older Adults. Arch Intern Med 2009;169:515-523.

20. Cohen RM, Franco RS, Khera PK, et al. Red cell life span heterogeneity in hematologically normal people is sufficient to alter HbAlc. Blood 2008;112:4284-4291.

21. Ladyzynski P, Wojcicki JM, Bak M, et al. Validation of hemoglobin glycation models using glycemia monitoring in vivo and culturing of erythrocytes in vitro. Ann Biomed Eng 2008;36:1188-1202.

22. Bunn HF, Gabbay KH, Gallop PM. GLYCOSYLATION OF HEMOGLOBIN - RELEVANCE TO DIABETES-MELLITUS. Science 1978;200:21-27.

23. Gram-Hansen P, Mourits-Andersen HT, Eriksen JE, et al. Glycosylated Hemoglobin (HbAlc) as an Index of the Age of the Erythrocyte Population in Non-Diabetic Patients. Eur J Haematol 1990;44:201-203.

24. Jansen H, Stolk RP, Nolte IM, et al. Determinants of HbAlc in nondiabetic Dutch adults: genetic loci and clinical and lifestyle parameters, and their interactions in the lifelines cohort study. Journal of Internal Medicine 2013;273:283-293.

25. Ladyzynski P, Wojcicki JM, Bak MI, et al. Hemoglobin Glycation Rate Constant in Non-diabetic Individuals. Ann Biomed Eng 2011;39:2721-2734.

26. Leslie RDG, Cohen RM. Biologic variability in plasma glucose, hemoglobin A1c, and advanced glycation end products associated with diabetes complications. Journal of diabetes science and technology 2009;3:635-643.

27. Mock DM, Widness JA, Veng-Pedersen P, et al. Measurement of Posttransfusion Red Cell Survival With the Biotin Label. Transf Med Rev 2014;28:114-125.

28. G. E, J.G. S, M. P, et al. Red cell distribution width, haemoglobin A1c and incidence of diabetes mellitus. Journal of Internal Medicine 2014.

29. Veeranna V, Zalawadiya SK, Panaich SS, et al. The Association of Red Cell Distribution Width with Glycated Hemoglobin among Healthy Adults without Diabetes Mellitus. Cardiology 2012;122:129-132.

30. Piva E, Brugnara C, Chiandetti L, et al. Automated reticulocyte counting: state of the art and clinical applications in the evaluation of erythropoiesis. Clinical Chemistry and Laboratory Medicine 2010;48:1369-1380.

31. Lozoff B, Jimenez E, Wolf AW. Long-Term Developmental Outcome of Infants with Iron-Deficiency. N Engl J Med 1991;325:687-694.

32. Rockey DC, Cello JP. Evaluation of the Gastrointestinal-Tract in Patient with Iron-Deficiency Anemia. N Engl J Med 1993;329:1691-1695.

**Claims**

1. A method for evaluating a risk of death within 2 years for a subject:

    measuring, using a hematology analyzer, a complete blood count (CBC) of a blood sample comprising a population of RBCs from a subject;
    estimating a normalized clearance volume of the population of the RBCs based on the CBC, the normalized clearance volume being indicative of a volume below which clearance occurs for most of the population of RBCs;
    comparing the normalized clearance volume to a reference value;
    determining the risk of death for the subject within 2 years, wherein the risk of death is high if the normalized clearance volume is below the reference value, and the risk of death is low if the normalized clearance volume is above the reference value; and
    selecting the subject for diagnostic workup if the risk of death is high.

2. The method of claim 1, wherein said estimating the normalized clearance volume of the population of the RBCs based on the CBC further comprises determining parameters indicative of each of:

    a magnitude of variation in a rate of hemoglobin content reduction ($D_h$);
    a magnitude of variation in a rate of RBC volume reduction ($D_v$);
    an average rate of slow-phase RBC volume and hemoglobin content reduction ($\alpha$);
    an average rate of fast-phase RBC volume reduction ($\beta_v$); and
    an average rate of fast-phase RBC hemoglobin content reduction ($\beta_h$).

3. The method of claim 1, wherein said estimating the normalized clearance volume comprises:

    computing a mean corpuscular hemoglobin concentration (MCHC) line from an MCHC of the CBC; and
    computing a probability of clearance of an RBC of the population of the RBCs based on the MCHC line.

4. The method of claim 1, wherein:

    said measuring the CBC comprises measuring a volume and hemoglobin content of the RBC, and
    the probability of clearance is a function of the volume and the hemoglobin content.

5. The method of claim 1, wherein the reference value is between 0.77 and 0.79.

6. A non-transitory machine-readable storage device storing computer program instructions executable by a computer system to cause the computer system to perform operations of the method of any one of claims 1 to 5.

**Patentansprüche**

1. Verfahren zum Einschätzen des Todesrisikos innerhalb von 2 Jahren für einen Probanden:

   Messen, mittels eines Hämatologie-Analysators, eines Blutstatus (CBC, Complete Blood Count) einer Blutprobe, umfassend eine Population von roten Blutkörperchen (RBC, Red Blood Cells), von einem Probanden;
   Einschätzen eines normalisierten Clearance-Volumens der Population der RBCs basierend auf dem CBC, wobei das normalisierte Clearance-Volumen indikativ für ein Volumen ist, unterhalb dessen eine Clearance für den größten Teil der Population von RBCs erfolgt;
   Vergleichen des normalisierten Clearance-Volumens mit einem Referenzwert;
   Bestimmen des Todesrisikos für den Probanden innerhalb von 2 Jahren, wobei das Todesrisiko hoch ist, wenn das normalisierte Clearance-Volumen unter dem Referenzwert liegt, und wobei das Todesrisiko gering ist, wenn das normalisierte Clearance-Volumen über dem Referenzwert liegt; und
   Auswählen des Probanden für eine diagnostische Abklärung, wenn das Todesrisiko hoch ist.

2. Verfahren nach Anspruch 1, wobei das Einschätzen des normalisierten Clearance-Volumens der Population der RBCs basierend auf dem CBC ferner umfasst Parameter zu bestimmen, die indikativ sind für jedes von:

   einer Größenordnung der Variation einer Rate der Verringerung des Hämoglobingehalts ($D_h$);
   eine Größenordnung der Variation einer Rate der Verringerung des RBC-Volumens ($D_v$);
   eine mittlere Rate einer langphasigen Verringerung des RBC-Volumens und des Hämoglobin-Gehalts (a);
   eine mittlere Rate einer kurzphasigen Verringerung des RBC-Volumens ($\beta_v$); und
   eine mittlere Rate einer kurzphasigen Verringerung des RBC-Hämoglobingehalts ($\beta_h$);

3. Verfahren nach Anspruch 1, wobei das Einschätzen des normalisierten Clearance-Volumens umfasst:

   Berechnen einer Linie der mittleren korpuskulären Hämoglobin-Konzentration (MCHC, Mean Corpuscular Hemoglobin Concentration) aus einer MCHC des CBC; und
   Berechnen einer Wahrscheinlichkeit der Clearance eines RBC der Population der RBCs basierend auf der MCHC-Linie.

4. Verfahren nach Anspruch 1, wobei:

   das Messen des CBC umfasst, ein Volumen und einen Hämoglobingehalt des RBC zu messen, und
   die Wahrscheinlichkeit der Clearance eine Funktion von Volumen und Hämoglobingehalt ist.

5. Verfahren nach Anspruch 1, wobei der Referenzwert zwischen 0,77 und 0,79 liegt.

6. Nichttransitorisches, maschinenlesbares Speichermedium, das Computerprogrammbefehle speichert, die von einem Computersystem ausführbar sind, um das Computersystem zu veranlassen, Operationen des Verfahrens nach einem der Ansprüche 1 bis 5 durchzuführen.


**Revendications**

1. Procédé d'évaluation d'un risque de décès à deux ans pour un sujet :

   mesurer, en utilisant un analyseur hématologique, une formule sanguine complète (FSC) d'un échantillon de sang comprenant une population de globules rouges (GR) provenant d'un sujet ;
   estimer un volume de clairance normalisé de la population des GR sur la base de la FSC, le volume de clairance normalisé étant représentatif d'un volume au-dessous duquel la clairance se produit pour la majeure partie de la population de GR ;
   comparer le volume de clairance normalisé à une valeur de référence ;
   déterminer le risque de décès pour le sujet à deux ans, le risque de décès étant élevé si le volume de carence normalisé est inférieur à la valeur de référence, et le risque de décès étant faible si le volume de clairance normalisé est supérieur à la valeur de référence ; et
   sélectionner le sujet pour un bilan diagnostique si le risque de décès est élevé.

**2.** Procédé de de la revendication 1, dans lequel ladite estimation du volume de clairance normalisé de la population des GR sur la base de la FSC comprend en outre la détermination de paramètres représentatifs de chacun des éléments suivants :

une amplitude de variation d'une vitesse de réduction de la teneur en hémoglobine ($D_h$) ;
une amplitude de variation d'une vitesse de réduction du volume de GR ($D_v$) ;
une vitesse moyenne de réduction du volume de GR et de la teneur en hémoglobine en phase lente (a) ;
une vitesse moyenne de réduction du volume de GR en phase rapide ($\beta_v$) ; et
une vitesse moyenne de réduction de la teneur en hémoglobine des GR en phase rapide ($\beta_h$).

**3.** Procédé de la revendication 1, dans lequel ladite estimation du volume de clairance normalisé comprend :

le calcul d'une ligne de la concentration corpusculaire moyenne en hémoglobine (CCMH) à partir d'une CCMH de la FSC ; et
le calcul d'une probabilité de clairance d'un GR de la population des GR sur la base de la ligne de CCMH.

**4.** Procédé de la revendication 1, dans lequel :

ladite mesure de la FSC comprend la mesure d'un volume et d'une teneur en hémoglobine des GR, et
la probabilité de clairance est une fonction du volume et de la teneur en hémoglobine.

**5.** Procédé de la revendication 1, dans lequel la valeur de référence se situe entre 0,77 et 0,79.

**6.** Dispositif de stockage non transitoire lisible par machine stockant des instructions de programme informatique exécutables par un système informatique pour conduire le système informatique à effectuer des opérations du procédé de l'une quelconque des revendications 1 à 5.

FIG. 1

FIG. 2

FIG. 3

FIG. 4

FIG. 5A

FIG. 5B

FIG. 5C

FIG. 6A

FIG. 6B

FIG. 6C

FIG. 6D

FIG. 6E

FIG. 7

FIG. 8A

FIG. 8B

FIG. 8C

FIG. 9A

FIG. 9B

FIG. 9C

FIG. 9D

FIG. 10

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012037524 A
- US 20110178716 A
- US 20110164803 A
- US 20110149061 A
- US 20110077871 A
- US 20110070606 A
- US 20110070210 A
- US 5017497 A
- US 5266269 A
- US 5378633 A
- US 5631165 A
- US 5812419 A
- US 6228652 B
- US 6524858 B
- US 6320656 B
- US 7324194 B
- US 7981681 B
- US 20080153170 A
- US 20080158561 A
- US 20080268494 A

**Non-patent literature cited in the description**

- **HARSH H. PATEL et al.** *American Journal of Hematology,* 01 May 2015, vol. 90 (5), 422-428
- **HOME BD.** A Changing Focus on the Red Cell Distribution Width: Why Does It Predict Mortality and Other Adverse Medical Outcomes?. *Cardiology,* 2012, vol. 122, 213-215
- **FELKER GM ; ALLEN LA ; POCOCK SJ et al.** Red cell distribution width as a novel prognostic marker in heart failure - Data from the CHARM program and the Duke Databank. *J Am Coll Cardiol,* 2007, vol. 50, 40-47
- **PERLSTEIN TS ; WEUVE J ; PFEFFER MA et al.** Red Blood Cell Distribution Width and Mortality Risk in a Community-Based Prospective Cohort. *Arch Intern Med,* 2009, vol. 169, 588-594
- **ANDERSON JL ; RONNOW BS ; HOME BD et al.** Usefulness of a complete blood count-derived risk score to predict incident mortality in patients with suspected cardiovascular disease. *Am J Cardiol,* 2007, vol. 99, 169-174
- **SPELL DW ; JONES DV ; HARPER WF et al.** The value of a complete blood count in predicting cancer of the colon. *Cancer Detect Prev,* 2004, vol. 28, 37-42
- **PASCUAL-FIGAL DA ; BONAQUE JC ; MANZANO-FERNANDEZ S et al.** Red blood cell distribution width predicts new-onset anemia in heart failure patients. *Int J Cardiol,* 2012, vol. 160, 196-200
- **ALLEN LA ; FELKER GM ; MEHRA MR et al.** Validation and Potential Mechanisms of Red Cell Distribution Width as a Prognostic Marker in Heart Failure. *J Card Fail,* 2010, vol. 16, 230-238
- **HOME BD.** The Red Cell Distribution Width: What Is Its Value for Risk Prognostication and for Understanding Disease Pathophysiology?. *Cardiology,* 2011, vol. 119, 140-141
- **HIGGINS JM ; MAHADEVAN L.** Physiological and pathological population dynamics of circulating human red blood cells. *Proc Natl Acad Sci U S A,* 2010, vol. 107, 20587-20592
- **WILLEKENS FLA ; WERRE JM ; GROENEN-DOP YAM et al.** Erythrocyte vesiculation: a self-protective mechanism?. *Br J Haematol,* 2008, vol. 141, 549-556
- **WILLEKENS FL ; ROERDINKHOLDER-STOEL-WINDER B ; GROENEN-DOPP YA et al.** Hemoglobin loss from erythrocytes in vivo results from spleen-facilitated vesiculation. *Blood,* 2003, vol. 101, 747-751
- **WAUGH RE ; NARLA M ; JACKSON CW et al.** Rheologic Properties of Senescent Erythrocytes - Loss of Surface-Area and Volume with Red-Blood-Cell Age. *Blood,* 1992, vol. 79, 1351-1358
- **FRANCO RS ; PUCHULU-CAMPANELLA ME ; BARBER LA et al.** Changes in the properties of normal human red blood cells during in vivo aging. *Am J Hematol,* 2013, vol. 88, 44-51
- **FRANCO RS.** The measurement and importance of red cell survival. *Am J Hematol,* 2009, vol. 84, 109-114
- **GIFFORD SC ; DERGANC J ; SHEVKOPLYAS SS et al.** A detailed study of time-dependent changes in human red blood cells: from reticulocyte maturation to erythrocyte senescence. *Br J Haematol,* 2006, vol. 135, 395-404

- **WILLEKENS FLA ; ROERDINKHOLDER-STOEL-WINDER B ; GROENEN-DOPP YAM et al.** Hemoglobin loss from erythrocytes in vivo results from spleen-facilitated vesiculation. *Blood,* 2003, vol. 101, 747-751
- **BEUTLER E ; WAALEN J.** The definition of anemia: what is the lower limit of normal of the blood hemoglobin concentration?. *Blood,* 2006, vol. 107, 1747-1750
- **DONOFRIO G ; CHIRILLO R ; ZINI G et al.** Simultaneous Measurement Of Reticulocyte And Red-Blood-Cell Indexes In Healthy-Subjects And Patients With Microcytic And Macrocytic ANEMIA. *Blood,* 1995, vol. 85, 818-823
- **PATEL KV ; FERRUCCI L ; ERSHLER WB et al.** Red Blood Cell Distribution Width and the Risk of Death in Middle-aged and Older Adults. *Arch Intern Med,* 2009, vol. 169, 515-523
- **COHEN RM ; FRANCO RS ; KHERA PK et al.** Red cell life span heterogeneity in hematologically normal people is sufficient to alter HbAlc. *Blood,* 2008, vol. 112, 4284-4291
- **LADYZYNSKI P ; WOJCICKI JM ; BAK M et al.** Validation of hemoglobin glycation models using glycemia monitoring in vivo and culturing of erythrocytes in vitro. *Ann Biomed Eng,* 2008, vol. 36, 1188-1202
- **BUNN HF ; GABBAY KH ; GALLOP PM.** GLYCOSYLATION OF HEMOGLOBIN - RELEVANCE TO DIABETES-MELLITUS. *Science,* 1978, vol. 200, 21-27
- **GRAM-HANSEN P ; MOURITS-ANDERSEN HT ; ERIKSEN JE et al.** Glycosylated Hemoglobin (HbAlc) as an Index of the Age of the Erythrocyte Population in Non-Diabetic Patients. *Eur J Haematol,* 1990, vol. 44, 201-203
- **JANSEN H ; STOLK RP ; NOLTE IM et al.** Determinants of HbAlc in nondiabetic Dutch adults: genetic loci and clinical and lifestyle parameters, and their interactions in the lifelines cohort study. *Journal of Internal Medicine,* 2013, vol. 273, 283-293
- **LADYZYNSKI P ; WOJCICKI JM ; BAK MI et al.** Hemoglobin Glycation Rate Constant in Non-diabetic Individuals. *Ann Biomed Eng,* 2011, vol. 39, 2721-2734
- **LESLIE RDG ; COHEN RM.** Biologic variability in plasma glucose, hemoglobin A1c, and advanced glycation end products associated with diabetes complications. *Journal of diabetes science and technology,* 2009, vol. 3, 635-643
- **MOCK DM ; WIDNESS JA ; VENG-PEDERSEN P et al.** Measurement of Posttransfusion Red Cell Survival With the Biotin Label. *Transf Med Rev,* 2014, vol. 28, 114-125
- **G. E ; J.G. S ; M. P et al.** Red cell distribution width, haemoglobin A1c and incidence of diabetes mellitus. *Journal of Internal Medicine,* 2014
- **VEERANNA V ; ZALAWADIYA SK ; PANAICH SS et al.** The Association of Red Cell Distribution Width with Glycated Hemoglobin among Healthy Adults without Diabetes Mellitus. *Cardiology,* 2012, vol. 122, 129-132
- **PIVA E ; BRUGNARA C ; CHIANDETTI L et al.** Automated reticulocyte counting: state of the art and clinical applications in the evaluation of erythropoiesis. *Clinical Chemistry and Laboratory Medicine,* 2010, vol. 48, 1369-1380
- **LOZOFF B ; JIMENEZ E ; WOLF AW.** Long-Term Developmental Outcome of Infants with Iron-Deficiency. *N Engl J Med,* 1991, vol. 325, 687-694
- **ROCKEY DC ; CELLO JP.** Evaluation of the Gastrointestinal-Tract in Patient with Iron-Deficiency Anemia. *N Engl J Med,* 1993, vol. 329, 1691-1695